# EUROPEAN PATENT SPECIFICATION

(11) **EP 3 727 494 B1**
(45) Date of publication and mention of the grant of the patent: **04.09.2024**
(21) Application number: 18829758.4
(22) Date of filing: 11.12.2018
(51) Int. Cl.: A61M 1/36

(54) **DIALYSIS SYSTEM HAVING CARBON DIOXIDE GENERATION AND PRIME**
DIALYSESYSTEM MIT KOHLENDIOXIDERZEUGUNG ZUM VORFÜLLEN
SYSTÈME POUR LA DIALYSE AVEC GÉNÉRATION DE CO2 POUR AMORCER

(30) Priority: 21.12.2017 SE 1751598
(43) Date of publication of application: 28.10.2020
(73) Proprietor: Gambro Lundia AB, 226 43 Lund (SE)
(72) Inventor: HOBRO, Sture, 226 47 LUND (SE); STERNBY, Jan, 222 36 LUND (SE); LINDEN, Torbjörn, 370 23 HASSLÖ (SE); JANSSON, Olof, 235 38 Vellinge (SE)
(74) Representative: Ponzellini, Gian-Marco
(86) International application number: PCT/EP2018/084368
(87) International publication number: WO 2019/121167

(56) References cited:
- US-A1- 2005 118 059
- US-A1- 2014 216 250

## Description

### BACKGROUND

The present disclosure relates generally to medical delivery. More specifically, the present disclosure relates to the priming of extracorporeal therapy machines.

Due to various causes, a person's renal system may fail. Renal failure produces several physiological derangements. It is no longer possible to balance water and minerals or to excrete daily metabolic load. Toxic end products of metabolism (urea, creatinine, uric acid, and others) may accumulate in blood and tissue.

Kidney failure and reduced kidney function have been treated with dialysis. Dialysis removes waste, toxins and excess water from the body that normal functioning kidneys would otherwise remove. Dialysis treatment for replacement of kidney functions is critical to many people because the treatment is life saving.

One type of kidney failure therapy is Hemodialysis ("HD"), which in general uses diffusion to remove waste products from a patient's blood. A diffusive gradient occurs across the semi-permeable dialyzer between the blood and an electrolyte solution called dialysate or dialysis fluid to cause diffusion.

Hemofiltration ("HF") is an alternative renal replacement therapy that relies on a convective transport of toxins from the patient's blood. HF is accomplished by adding substitution or replacement fluid to the extracorporeal circuit during treatment (typically ten to ninety liters of such fluid). The substitution fluid and the fluid accumulated by the patient in between treatments is ultrafiltered over the course of the HF treatment, providing a convective transport mechanism that is particularly beneficial in removing middle and large molecules (in hemodialysis there is a small amount of waste removed along with the fluid gained between dialysis sessions, however, the solute drag from the removal of that ultrafiltrate is not enough to provide sufficient convective clearance).

Hemodiafiltration ("HDF") is a treatment modality that combines convective and diffusive clearances. HDF uses dialysis fluid flowing through a dialyzer, similar to standard hemodialysis, to provide diffusive clearance. In addition, substitution solution is provided directly to the extracorporeal circuit, making increased ultrafiltration possible, which in turn yields convective clearance.

Most HD (HF, HDF) treatments occur in centers. A trend towards home hemodialysis ("HHD") exists today in part because HHD may be performed daily, offering therapeutic benefits over in-center hemodialysis treatments, which occur typically bi- or tri-weekly. Studies have shown that more frequent treatments remove more toxins and waste products than treatments occurring less frequently, but lasting longer. A patient receiving more frequent treatments does not experience as much of a down cycle as does an in-center patient, who has built-up two or three day's worth of toxins prior to a treatment. In certain areas, the closest dialysis center may be many miles from the patient's home causing door-to-door treatment time to consume a large portion of the day. HHD may take place overnight or during the day while the patient relaxes, works or is otherwise productive. <INSERT PAGE 2A>

In any of the above modalities, it is necessary to prime the associated equipment prior to running a treatment on a patient. Priming typically involves displacing air with a physiologically compatible solution such as dialysis fluid or saline. Air may however be difficult to remove from certain areas of a blood circuit, such as within the membranes of a dialyzer. Pockets of air may remain even after being flushed with a solution.

An improved priming system and method is needed accordingly.

### SUMMARY

The examples described herein disclose automated systems and methods applicable, for example, to fluid delivery for: plasmapherisis, hemodialysis ("HD"), hemofiltration ("HF") hemodiafiltration ("HDF"), continuous renal replacement therapy ("CRRT"), apheresis, autotransfusion, hemofiltration for sepsis, and extracorporeal membrane oxygenation ("ECMO") therapies (extracorporeal therapies). Any of the extracorporeal therapies may require that the blood circuit including a blood filter (dialyzer, hemofilter, etc.) be primed prior to being connected to the patient. The present <Document US2014216250A1 discloses a degassing module for removal of air and other gases during operation of a medical therapy device that delivers any one of hemodialysis, hemodiafiltration and hemofiltration. The degassing module has a flow-through first chamber that has a hydrophobic vent membrane that has an exterior and interior side forming a portion of the flow-through chamber.

Document US2005118059A1 discloses an extracorporeal blood circuit for use with a venous return line and an arterial line coupled to a patient. The extracorporeal blood circuit can include a venous air removal device coupled to the venous return line. The venous air removal device can perform an active air removal function. The extracorporeal blood circuit can include a sensor that determines a blood level in the venous air removal device, a purge line coupled to the venous air removal device, and a controller connected to the sensor. The controller can cause the venous air removal device to perform the active air removal function through the purge line when the blood level is less than a threshold.> disclosure sets forth systems and methods that use carbon dioxide ("CO₂") gas to initially prime the blood circuit. CO₂ gas is better able than a priming fluid to dislodge air trapped in the membranes, and indeed within the pores of the membranes, of a blood filter. CO₂ gas may also be better at flushing air out of pockets located within other areas of the blood circuit, e.g., pockets created by the housing of a drip chamber of the blood circuit, or pockets created by the sealing of connectors to tubes (or the connectors themselves), or tubes to tubes, within the blood circuit. In short, CO₂ gas is better able to enter and flush small open spaces than is typical priming fluid.

There are two primary embodiments for performing the CO₂ gas prime of the present disclosure. In one primary embodiment, CO₂ gas is created by the system itself but outside of the extracorporeal or blood circuit. Here, CO₂ gas is transported into the blood circuit or set. In a second primary embodiment, CO₂ gas is carried by the fluid creating the CO₂ gas into the extracorporeal or blood circuit and released.

In the first primary embodiment, a CO₂ gas chamber may be provided, which receives the constituent fluids that mix together to form CO₂ gas and then stores at least a portion of the CO₂ gas. The constituent fluids that mix together to form CO₂ gas may include bicarbonate (e.g., from B-concentrate) and any one of: (i) a citric acid solution, (ii) an acetic acid solution (e.g., from A-concentrate), (iii) a lactic acid solution, (iv) a malic acid solution, (v) a hydrochloric ("HCl") acid solution, (vi) a phosphoric acid solution, (vii) any other biocompatible acid solution (e.g., an acid whose anion is present in a human body), and (v) blends thereof. The system in an embodiment uses A-concentrate and B-concentrate to make a dialysis fluid for treatment. If using A-concentrate and B-concentrate to also make the CO₂ gas generation fluid, then no additional source of acid is needed, however, the CO₂ gas generation fluid made from A-concentrate and B-concentrate will be formulated differently, e.g., have a different or lower pH, than the treatment fluid made from A-concentrate and B-concentrate.

If using citric acid solution, an acetic acid solution or any other acid solution other than A-concentrate, then a separate source of the additional acid is provided, then the CO₂ gas generation fluid will have a different constituent makeup than the dialysis treatment fluid. In any case, an accurate metering pump may be provided to pump, in any order, bicarbonate solution in a desired amount and any of the acid solutions in a desired amount into the gas chamber. The bicarbonate and acid mix to form CO₂ gas over the course of a short period of time.

A CO₂ gas line is provided in one embodiment to run from an upper portion of the CO₂ gas chamber to a desired gas injection location. The CO₂ gas line may be opened or closed during the formation of CO₂ gas (which happens very quickly, on the order of seconds) depending upon how much pressure the CO₂ gas line is rated to hold. The desired injection location may, for example, be the fresh dialysis fluid line leading to a port of the blood filter, be a substitution fluid line leading to the arterial or venous blood line, or be a line leading to a port of the machine for receiving the connected blood lines for priming. In any case, the desired injection location is positioned and arranged to deliver the CO₂ gas into the blood circuit.

Under certain circumstances, it may be required to lower the pressure downstream from the CO₂ gas chamber to help draw the CO₂ gas from the CO₂ priming fluid. The underpressure may be created for example by operating the blood pump in reverse to place the dialyzer and the CO₂ gas line leading to the dialyzer under a negative pressure. The blood pump may also be operated (e.g., multiple times in normal and reverse directions) during the CO₂ gas prime to help distribute the CO₂ gas to all portions of the blood circuit or set. The CO₂ gas during the CO₂ gas prime pushes air in one embodiment out of a vent provided for example with a venous air trap of the blood set. As shown below, an alternative to an underpressure, or perhaps in addition to the underpressure, the CO₂ priming fluid may be mixed or stirred to release the CO₂ gas.

In addition to, or alternatively to, the use of an underpressure to release CO₂ gas from the CO₂ gas generation fluid, the metering pump may also operate with a recirculation line that recirculates the CO₂ gas generation fluid to thereby stir and agitate the CO₂ gas generation fluid. Stirring and agitating the CO₂ gas generation fluid helps to release the CO₂ gas from the fluid. It is contemplated to stir and agitate the CO₂ priming fluid at a location elevationally below a location where the CO₂ gas is collected and then delivered to the blood set.

When the CO₂ gas prime has been completed, the system and method of the first primary embodiment may then perform an additional prime of the blood set using a priming fluid such as saline or dialysis fluid prepared for treatment. The priming fluid such as saline or dialysis fluid prepared for treatment may absorb the CO₂ gas and/or expel the gas from the blood circuit, e.g., via the vent of the venous air trap. The dual stage prime, in addition to the CO₂ gas prime, results in superior air removal.

The second primary embodiment, in which CO₂ gas is carried by the CO₂ gas generation fluid into the blood set where it is released, does not need a CO₂ gas chamber or separate metering pump. The CO₂ gas generation fluid may again be formed via a bicarbonate solution and any of the acid solutions discussed herein, e.g., (i) a citric acid solution, (ii) an acetic acid solution (e.g., from A-concentrate), (iii) a lactic acid solution, (iv) a malic acid solution, (v) a hydrochloric ("HCl") acid solution, (vi) a phosphoric acid solution, (vii) any other biocompatible acid solution, and (v) blends thereof. The CO₂ gas generation fluid may be delivered to the blood circuit via any of the entry locations discussed herein, e.g., at the dialyzer port, substitution port, or arterial and venous line connection port.

An underpressure may again be needed to separate the CO₂ gas from the CO₂ gas generation fluid. The underpressure may be created by running different pumps at different speeds and/or by subjecting a portion of a blood line for which CO₂ gas contact is desired to a negative or intake side of a pump, e.g., a peristaltic blood pump. The blood pump may again be run in normal and reverse directions multiple times to ensure that the CO₂ gas is separated effectively from the CO₂ gas generation fluid and distributed effectively to all portions of the blood set.

When the CO₂ gas prime of the second primary embodiment has been completed, the system and method of the present disclosure again perform an additional prime of the blood set using a priming fluid such as saline or dialysis fluid prepared for treatment. The priming fluid such as saline or dialysis fluid prepared for treatment may absorb the CO₂ gas and/or expel the gas from the blood circuit, e.g., via the vent of the venous air trap.

The extracorporeal therapy system of the invention is according to claims 1-12; a priming method for an extracorporeal therapy system of the invention is according to claims 13-15.

In light of the present disclosure it is therefore an advantage of the present disclosure to provide an extracorporeal therapy system and method having improved priming.

It is another advantage of the present disclosure to provide an extracorporeal therapy system and method capable of generating its own CO₂ gas for priming.

It is a further advantage of the present disclosure to provide a relatively inexpensive system and method to improve priming.

It is still another advantage of the present disclosure to provide a system and method to improve priming that is physiologically compatible.

It is still a further advantage of the present disclosure to provide a system and method to improve priming that is time efficient.

It is yet another advantage of the present disclosure to provide a system and method to improve priming that requires little or no additional setup.

Still another advantage of the present disclosure is to reduce the amount of typical priming fluid, e.g., saline, which reduces cost and fluid priming time.

The advantages discussed herein may be found in one, or some, and perhaps not all of the embodiments disclosed herein. Additional features and advantages are described herein, and will be apparent from, the following Detailed Description and the figures.

### BRIEF DESCRIPTION OF THE FIGURES

Fig. 1A is a schematic illustration of one system and method of the present disclosure of the present disclosure employing CO₂ gas generation and priming of the present disclosure.
Fig. 1B is a schematic illustration of an alternative version of the system of Fig. 1A and includes structure for stirring or agitating the CO₂ priming fluid to release CO₂ gas therefrom.
Fig. 1C is a schematic illustration of an alternative version for a blood circuit or set that may be used with the system of Figs. 1A and 1B and includes a pressure measurement line in the blood set for receiving air displaced by CO₂ gas.
Fig. 1D is a schematic illustration of the system of Fig. 1C showing a priming fluid sequence to purge and absorb CO₂ gas from the blood set before treatment.
Figs. 2A to 2D are schematic illustrations of another system and method of the present disclosure, employing one embodiment for CO₂ gas generation and priming of the present disclosure, which releases CO₂ gas from a CO₂ gas generation fluid within the blood circuit.
Figs. 3A to 3D are schematic illustrations of a further system and method of the present disclosure, employing another embodiment for CO₂ gas generation and priming of the present disclosure, which releases CO₂ gas from a CO₂ gas generation fluid within the blood circuit.
Figs. 4A and 4B are schematic illustrations of still another system and method of the present disclosure, employing another embodiment for CO₂ gas generation and priming of the present disclosure, which releases CO₂ gas from a CO₂ gas generation fluid within the blood circuit.

### DETAILED DESCRIPTION

The examples described herein are applicable to any medical fluid therapy system that delivers a medical fluid, such as blood, dialysis fluid, substitution fluid, purified or sterilized water, liquid concentrate, or an intravenous drug. The examples are particularly well suited for kidney failure therapies, such as all forms of hemodialysis ("HD"), plasmapherisis, hemofiltration ("HF") hemodiafiltration ("HDF"), continuous renal replacement therapy ("CRRT"), apheresis, autotransfusion, hemofiltration for sepsis, and extracorporeal membrane oxygenation ("ECMO") treatments, referred to herein collectively or generally individually as an extracorporeal therapy. Moreover, the systems and methods described herein may be used in clinical or home settings. Extracorporeal therapy system 10a in the examples below is described as a renal failure therapy system having a machine 12 that creates online dialysis fluid for treatment.

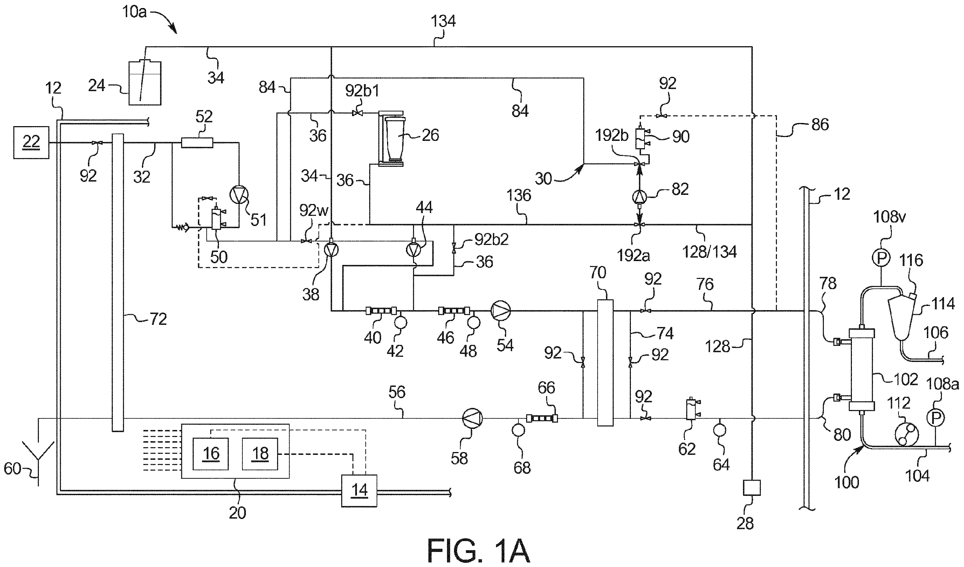

Referring now to Fig. 1A, one embodiment for a renal failure therapy system 10a employing an improved priming method or procedure of the present disclosure is illustrated. System 10a includes a machine 12 having an enclosure or housing. The housing of machine 12 holds the contents of a dialysis fluid circuit 30 described in detail below. The housing of machine 12 also supports a user interface 14, which allows a nurse or other operator to interact with system 10a. User interface 14 may have a monitor screen operable with a touch screen overlay, electromechanical buttons, e.g., membrane switches, or a combination of both. User interface 14 is in electrical communication with at least one processor 16 and at least one memory 18. At least one processor 16 and at least one memory 18 also electronically interact with, and where appropriate control, the pumps, valves and sensors described herein, e.g., those of dialysis fluid circuit 30. At least one processor 16 and at least one memory 18 are referred to collectively herein as a control unit 20. The dashed lines extending from control unit 20 are electrical or signal lines leading to and/or from pumps, valves, sensors, the heater and other electrical equipment of system 10a.

Dialysis fluid circuit 30 includes a purified water line 32, an A-concentrate line 34 and a bicarbonate B-concentrate line 36. Purified water line 32 receives purified water from a purified water device or source 22. The water may be purified using any one or more process, such as, reverse osmosis, carbon filtering, ultraviolet radiation, electrodeionization ("EDI"), and/or ultrafiltering.

An A-concentrate pump 38, such as a peristaltic or piston pump, pumps A-concentrate from an A-concentrate source 24 to mix with purified water from purified water line 32 via A-concentrate line 34. Conductivity cell 40 measures the conductive effect of the A-concentrate on the purified water, sends a signal to control unit 20, which uses the signal to properly proportion the A-concentrate by controlling A-concentrate pump 38. The A-conductivity signal may be temperature compensated via a reading from temperature sensor 42.

A B-concentrate pump 44, such as a peristaltic or piston pump, in the illustrated embodiment pumps purified water from purified water line 32, through B-concentrate line 36 and a B-concentrate source or bicarbonate cartridge 26 located along B-concentrate line 36, into a mixture of purified water and A-concentrate leaving conductivity sensor 40. Conductivity cell 46 measures the conductive effect of the B-concentrate on the purified water/A-concentrate mixture, sends a signal to control unit 20, which uses the signal to properly proportion the B-concentrate by controlling B-concentrate pump 44. The B-conductivity signal may also be temperature compensated via a reading from temperature sensor 48.

Purified water is degassed prior to receiving the concentrates, removing bubbles from the water. The water is degassed in a chamber 50 via a degassing pump 51, placed in fluid communication with degassing chamber 50. A heater 52 controlled by control unit 20 heats the purified water for treatment to body temperature, e.g., 37°C. The fluid exiting conductivity cell 46 is therefore freshly prepared dialysis fluid, properly degassed and heated, and suitable for sending to dialyzer 102 for treatment. Fresh dialysis fluid for a renal therapy treatment, such as hemodialysis, hemofiltration or hemodiafiltration, may be said to be made from a formula or at least one setpoint, e.g., conductivity setpoints for sensors 40 and 46, which is (are) specified according to a physiological constituency of the dialysis fluid that is suitable for treatment, e.g., suitable for the patient. The treatment formula or at least one setpoint may be different than ones described below to create dialysis fluid for priming, referred to herein as a priming fluid, wherein that dialysis fluid is formulated or regulated to create carbon dioxide ("CO₂") gas.

It is worth noting that priming blood set 100 is most often done using a priming fluid, which may be hung on a stand, and which is normally a saline solution. When priming using an online fluid (dialysis machine 12 itself produces the priming fluid), there are several options depending upon the type of machine 12. For example, the priming fluid may be dialysis fluid prepared for treatment, an A-concentrate based solution (possibly not the best alternative) or a sodium chloride fluid if machine 12 is able to separate a source of sodium from its other concentrates. Each of the examples listed above, including saline, is different than the CO₂ producing priming solutions of the present disclosure.

Fig. 1A further illustrates a fresh dialysis fluid pump 54, such as a gear pump, which delivers fresh dialysis fluid for treatment to dialyzer 102. Control unit 20 controls fresh dialysis fluid pump 54 to deliver fresh dialysis fluid to the dialyzer at a specified flowrate. A used dialysis fluid line 56 via a used dialysis fluid pump 58 returns used dialysis fluid from dialyzer 102 to a drain 60. Control unit 20 controls used dialysis fluid pump 58 to pull used dialysis fluid from dialyzer 102 at a specified flowrate. An air separator 62 separates air from the used dialysis fluid line 56. A pressure sensor 64 senses the pressure of used dialysis fluid line 56 and sends a corresponding pressure signal to control unit 20.

Conductivity cell 66 measures the conductivity of used fluid flowing through used dialysis fluid line 56 and sends a signal to control unit 20. The conductivity signal of cell 66 may also be temperature compensated via a reading from temperature sensor 68. A blood leak detector (not illustrated), such as an optical detector, looks for the presence of blood in used dialysis fluid line 56, e.g., to detect if a dialyzer membrane has a tear or leak. A heat exchanger 72 in the illustrated embodiment recoups heat from the used dialysis fluid exiting dialysis fluid circuit 30 to drain 60, preheating the purified water traveling towards heater 52 to conserve energy.

A fluid bypass line 74 allows fresh dialysis fluid to flow from fresh dialysis fluid line 76 to used dialysis fluid line 56 without contacting dialyzer 102. A fresh dialysis fluid tube 78 extends from machine 12 and carries fresh dialysis fluid from fresh dialysis fluid line 76 to dialyzer 102. A used dialysis fluid tube 80 also extends from machine 12 and carries used dialysis fluid from dialyzer 102 to used dialysis fluid line 56.

Dialysis circuit 30 also includes an ultrafiltration ("UF") system 70. UF system 70 monitors the flowrate of fresh dialysis fluid flowing to dialyzer 102 (and/or as substitution fluid flowing directly to the blood set 100) and used fluid flowing from dialyzer 102. UF system 70 may include fresh and used flow sensors, which send signals to control unit 20 indicative of the fresh and used dialysis fluid flowrates, respectively. Control unit 20 uses the signals to set used dialysis fluid pump 58 to pump faster than fresh dialysis fluid pump 54 by a predetermined amount to remove a prescribed amount of UF from the patient over the course of treatment.

An ultrafilter (not illustrated) may also be provided to further purify the fresh dialysis fluid before being delivered via dialysis fluid line 76 and fresh dialysis fluid tube 78 to dialyzer 102. Alternatively or additionally, one or more ultrafilter may be used to purify the fresh dialysis fluid from fresh dialysis fluid line 76 to the point where the fluid may be used as a substitution fluid to perform from pre- or post-dilution hemofiltration or hemodiafiltration.

System 10a provides plural valves 92, e.g., solenoid valves, each under the control of control unit 20 to selectively control a prescribed treatment. In particular, valve 92 in bypass line 74 selectively opens and closes the bypass line, e.g., to work with valve 92 of fresh dialysis fluid line 76 to set machine 12 into a bypass mode when, for example, the prepared dialysis fluid is incorrect in any way (composition, temperature). Bypass line valve 92 is accordingly used mainly for safety reasons. Valve 92 in fresh dialysis fluid line 76 selectively opens and closes the fresh dialysis fluid line. Valve 92 in used dialysis fluid line 56 selectively opens and closes the used dialysis fluid line. Valves 92 and 92w are located in purified water line 32 to selectively open and close the purified water line to purified water source 22, to deaerate the purified water, and to deliver the purified water for mixing with the concentrates. Multiple valves 92 are also provided to operate UF system 70.

Valves 92b1 and 92b2 in the illustrated embodiment may be used in a priming sequence for bicarbonate cartridge 26. In the illustrated embodiment, bicarbonate cartridge 26 holds a powder concentrate that is contacted with purified water, dissolving the bicarbonate powder to form a saturated concentrate. In one possible priming sequence, control unit 20 causes valves 92b1 and 92w to close and valve 92b2 to open. Fresh dialysis fluid pump 54 is operated, creating a sub-atmospheric pressure in the cartridge. Control unit 20 then opens valve 92b1 to introduce purified water. Cartridge 26 is primed and when the conductivity cell 46 senses a rise in conductivity, letting control unit 20 know that the priming sequence is finished and to close valve 92b2.

It should be appreciated that the dialysis fluid circuit 30 is simplified and may include other structure (e.g., more valves) and functionality not illustrated. Also, dialysis fluid circuit 30 illustrates one example of a hemodialysis ("HD") pathway. It is contemplated to provide one or more ultrafilter (not illustrated) in fresh dialysis fluid line 76 to create substitution fluid for hemofiltration ("HF"). It is also contemplated to provide one or more ultrafilter in one or more line(s) branching off of fresh dialysis fluid line 76 to create substitution fluid, in addition to the fresh dialysis fluid in line 76, for hemofiltration ("HF") or hemodiafiltration ("HDF").

Fig. 1A further illustrates one embodiment of a blood circuit or set 100 that may be used with machine 12 of system 10a. Blood circuit or set 100 includes a dialyzer 102 having many hollow fiber semi-permeable membranes, which separate dialyzer 102 into a blood compartment and a dialysis fluid compartment. The dialysis fluid compartment during treatment is placed in fluid communication with a distal end of fresh dialysis fluid tube 78 and a distal end of used dialysis fluid tube 80. For HF and HDF, a separate substitution tube, in addition to fresh dialysis fluid tube 78, is placed during treatment in fluid communication with one or both of arterial line 104 extending from an arterial access needle or cannula (not illustrated) or venous line 106 extending to a venous access needle or cannula (not illustrated). For HDF, dialysis fluid also flows through dialysis fluid tube 78 to dialyzer 102, while for HF, dialysis fluid flow through tube 78 is blocked.

An arterial pressure pod 108a may be placed upstream of blood pump 112 (such as a peristaltic or volumetric membrane pump), while venous line 106 includes a pressure pod 108v. Pressure pods 108a and 108v operate with blood pressure sensors (not illustrated) mounted on the housing of machine 12, which send arterial and venous pressure signals, respectively, to control unit 20. Venous line 106 includes a venous drip chamber 114, which removes air from the patient's blood before the blood is returned to the patient. Venous chamber 114 may be provided with a hydrophobic or hydrophilic vent 116.

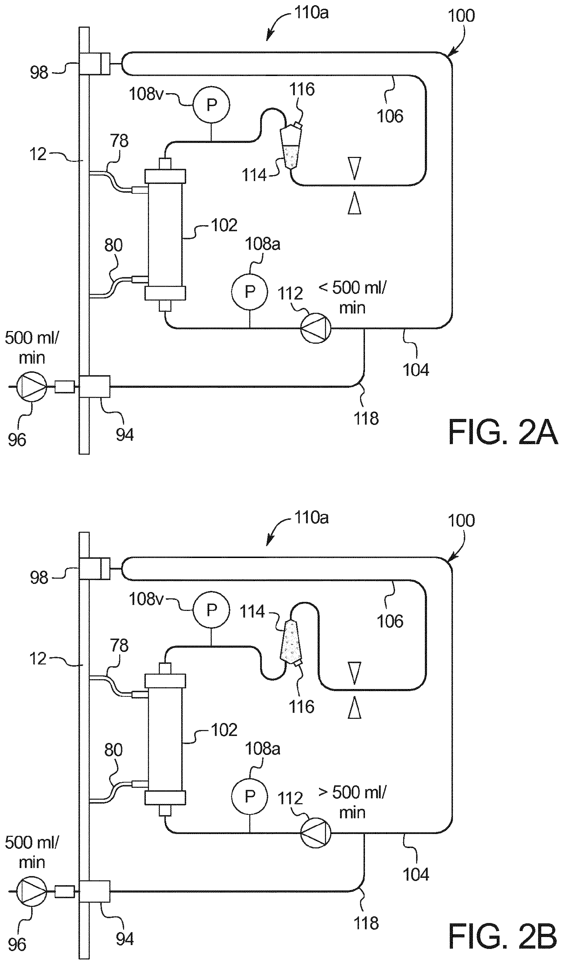

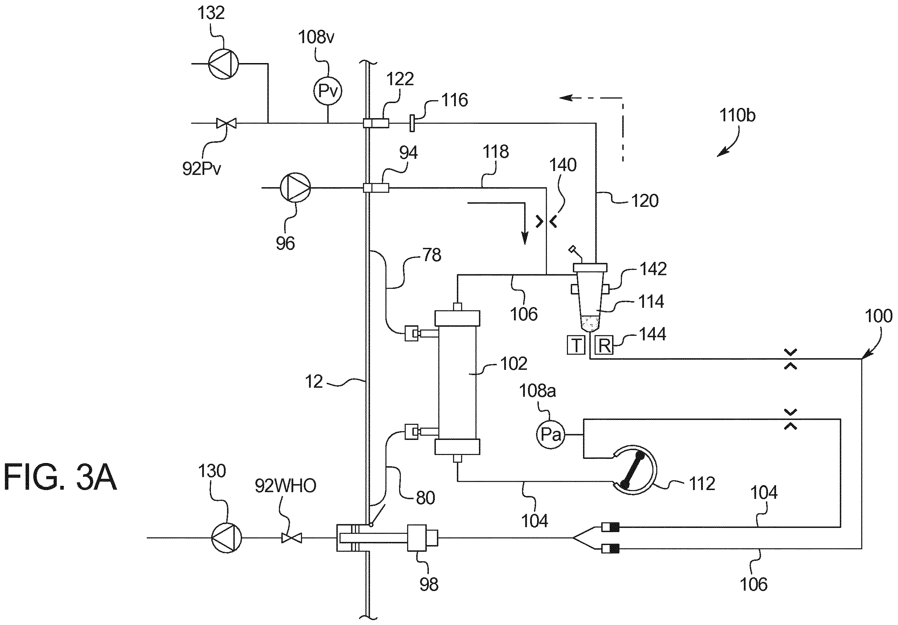

In the illustrated embodiment, arterial line 104 of blood circuit or set 100 is operated by blood pump 112, which is under the control of control unit 20 to pump blood at a desired flowrate. System 10a also provides multiple blood side electronic devices that send signals to and/or receive commands from control unit 20. For example, control unit 20 commands pinch clamps (Figs. 2A to 2D and clamp 140 in Figs. 3A to 3D) to selectively open or close arterial line 104 and/or venous line 106. A blood volume sensor ("BVS", not illustrated) may be located along arterial line 104 upstream of blood pump 112. An air detector (not illustrated) may be provided to look for air in venous blood line 106.

Fig. 1A further illustrates structure for enabling CO₂ gas to be generated in dialysis fluid circuit 30 and to be delivered to blood circuit 100 for priming purposes. In particular, dialysis fluid circuit 30 shows a bicarbonate priming line 136, running to a first three-way solenoid valve 192a under control of control unit 20. A metering pump 82 under control of control unit 20, such as a volumetric or membrane pump, or perhaps an accurately controlled peristaltic pump or gear pump is located between first three-way solenoid valve 192a and a second three-way solenoid valve 192b under control of control unit 20.

The third leg of first three-way solenoid valve 192a is connected fluidly to a citric acid line 128 leading to a citric acid source 28. Citric acid for source 28 may for example be provided in a concentration of 20% to 60% by volume and in one preferred embodiment 50% by volume. The upper citric acid limit is affected by the need to avoid the formation of precipitation. An upper limit of 50% to 60% should avoid such formation. To avoid shipping water and to avoid replacing the concentrate as much as possible, the concentration should be as high as possible. Lower citric acid concentrations are otherwise theoretically useable as long as the citric acid level is not so low that bacteria is allowed to form.

The second and third legs of second three-way solenoid valve 192b in the illustrated embodiment are connected fluidly to a gas generation chamber 90 and a return line 84. Gas generation chamber 90 may be made of metal or plastic as desired and includes an inlet for receiving fluids from metering pump 82 and an outlet in fluid communication with a CO₂ gas line 86, which is connected fluidly to fresh dialysis fluid line 76. Return line 84 returns fluids used to make CO₂ gas to a point upstream of dialysis fluid pump 54, e.g., to bicarbonate B-concentrate line 36. Return line 84 allows control unit 20 to verify via the conductivity sensors that bicarbonate concentrate is present at valve 192b before opening the valve to fill gas generation chamber 90. Without such verification, the system may instead first fill gas generation chamber 90 with purified water, possibly causing an incorrect amount of concentrate to be delivered to the chamber. Return line 84 helps to ensure that bicarbonate priming line 136 is primed with bicarbonate fluid prior to infusing fluid into gas generation chamber 90.

Degassing pump 51 under control of control unit 20 is in one embodiment used mainly to create low pressure in a degassing loop that includes heater 52 and degassing chamber 50 to degas the water for priming and treatment. To make CO₂ gas generation fluid in an embodiment, system 10a may be currently producing dialysis fluid for treatment, however, it is not a necessity that system 10a is doing so. Bicarbonate cartridge 26 should be primed in any case however. As illustrated, return line 84 is positioned and arranged such the control unit 20 advantageously does not have to stop B-concentrate pump 44 to provide bicarbonate solution for CO₂ gas generation. Control unit 20 causes metering pump 82 to pump through or from bicarbonate cartridge 26 (dry or liquid source) to valve 192b and further into return line 84. When the bicarbonate solution reaches a point just ahead of valve 92w, the bicarbonate solution may begin to enter the main fresh dialysis fluid line 76, so that the bicarbonate solution is sensed by conductivity sensors 40 and 46 feeding corresponding signals to control unit 20.

Control unit 20 then causes valve 192b to switch, so that the bicarbonate solution enters gas generation chamber 90. Control unit 20 may cause A- and B-concentrate pumps 38 and 44 to run at fixed, e.g., slower, operating speeds, so that concentrate pumps 38 and 44 continue to prepare dialysis fluid for treatment (assuming dialysis fluid is being prepared), while preparing B-concentrate for CO₂ gas generation. In an embodiment, the dialysis fluid for treatment is used at this time (prior to treatment) to prime fresh dialysis fluid line 76 and used dialysis fluid line 56.

When a desired amount of bicarbonate solution has been added to gas generation chamber 90, the acid step is started, e.g., adding either citric acid from citric acid source 28, A-concentrate from A-concentrate source 24 or some other acid containing fluid. Dependent on which acid fluid is used, the acid pumping sequence may vary. Using citric acid from source 28 is straight forward in the illustrated embodiment, namely, control unit 20 causes metering pump 82 to pull a desired amount of citric acid from source 28 into gas generation chamber 90. If A-concentrate from source 24 is used instead, control unit 20 performs a priming sequence similar to that for the B-concentrate, where A-concentrate is used to prepare dialysis solution for treatment and A-concentrate for CO₂ gas generation simultaneously (e.g., where the dialysis fluid for treatment is used at this time (prior to treatment) to prime fresh dialysis fluid line 76 and used dialysis fluid line 56). Here again, control unit 20 may cause A- and B-concentrate pumps 38 and 44 to run at fixed, e.g., slower, operating speeds. In one embodiment, control unit 20 ensures that an acid solution is provided at the entrance of valve 192b. Conductivity sensors 40 and 46 operating with control unit 20 are used again to detect when the acid solution has entered main fresh dialysis fluid line 76. The acid solution is then metered into gas generation chamber 90 in the same manner as described above for the bicarbonate solution.

Control unit may alternatively cause the A- and B-concentrate pumps 38 and 44 to be actively controlled during the A- and B-concentrate delivery for CO₂ gas generation. Being actively controlled in an embodiment means the speed of pumps 38 and 44 may change due to feedback presented to control unit 20. Here, control unit 20 may be able to detect when A- and B-concentrate delivery for CO₂ gas generation has been completed by noting a change to the speed or the pump revolutions over time for A- or B-concentrate pumps 38 and 44.

Next, with the port to bicarbonate priming line 136 of first three-way solenoid valve 192a closed and its other two ports open, the port to return line 84 of second three-way solenoid valve 192b closed and its other two ports open, and valve 92 in CO₂ gas line 86 open or closed as desired, metering pump 82 pumps a specified amount of citric acid (e.g., in a specified concentration) from source 28 via citric acid line 128 into gas generation chamber 90 to mix with the bicarbonate solution.

With the pathway through second three-way solenoid valve 192b between metering pump 82 and return line 84 closed, and valve 92 in CO₂ gas line 86 open or closed as desired, the citric acid and bicarbonate solution may be allowed to mix for a specified period of time, e.g., a short period of time such as from a few seconds to 120 seconds, to produce a volume of CO₂ gas that will pressurize inside gas generation chamber 90. Once the specified CO₂ gas production period has ended, and with the most downstream valve 92 in fresh dialysis fluid line 76 closed and the pathway through second three-way solenoid valve 192b between metering pump 82 and return line 84 closed, control unit 20 in one embodiment causes valve 92 in CO₂ gas line 86 to open (if previously closed), allowing the pressurized CO₂ gas inside chamber 90 to flow through CO₂ gas line 86, a distal portion of dialysis fluid line 76, and fresh dialysis fluid tube 78 into blood circuit 100 via dialyzer 102. If valve 92 in CO₂ gas line 86 has instead been open during CO₂ gas formation, then the CO₂ gas migrates naturally along gas line 86.

In one embodiment, control unit 20 causes valve 92 in CO₂ gas line 86 to be open during CO₂ gas production (to allow for CO₂ gas formation and the use of lower pressure components). In an alternative embodiment, control unit 20 may cause valve 92 in CO₂ gas line 86 to be closed during CO₂ generation. Here, a pressure gauge in signal communication with control unit 20 may be located appropriately, e.g., along gas line 86, and/or a pressure relief valve set at an appropriate operating limit may be provided along gas line 86 to ensure that pressurized CO₂ does not exceed a limit set for gas line 86.

During the CO₂ priming of the present disclosure, arterial line 104 and venous line 106 are connected together at their distal ends, forming a loop that traps the CO₂ gas within blood circuit 100. Hydrophobic or hydrophilic vent 116 located on venous drip chamber 114 allows the heavier CO₂ gas to push the lighter air in blood circuit 100 out of the circuit. CO₂ gas is better able than a priming fluid to dislodge air trapped in the membranes, and indeed within the pores of the membranes, of dialyzer 102. CO₂ gas may also be better at flushing air out of pockets located within blood circuit 100, e.g., pockets created by the housing of dialyzer 102, pockets created by the housing of venous drip chamber 114, or even pockets created by the sealing of connectors to tubes (or the connectors themselves), or tubes to tubes, within blood circuit 100. In short, CO₂ gas is better able to enter and flush small open spaces than is typical priming fluid.

In an alternative embodiment, arterial line 104 and venous line 106 may be connected to each other via a disposable piece (not illustrated) containing the venting function (e.g., venting air to atmosphere or trapping the air) instead of incorporating the venting function at venous drip chamber 114. In a further alternative embodiment, the air and CO₂ gas are removed from blood set 100 via a pressure measurement line 120 illustrated below in connection with Fig. 3A.

In an embodiment, the bicarbonate solution and citric acid reaction within chamber 90 creates a volume of CO₂ gas, which is a multiple (e.g., 4X) of the total volume of blood circuit 100, including dialyzer 102 and drip chamber 114. Thus if the total volume of blood circuit 100 is about two-hundred fifty milliliters ("mL"), the volume of CO₂ created within chamber 90 and delivered to blood circuit 100 may be about one liter. Given the ability of CO₂ gas to reach and purge small air pockets, and the multiple blood circuit volume's worth of CO₂ gas produced for priming, it is believed that virtually all of the air will be removed from blood circuit 100 via the CO₂ gas prime.

It should be appreciated that blood circuit 100 and in particular dialyzer 102 should be substantially dry or as dry as possible (in one embodiment completely dry) for the CO₂ gas prime. Most dialyzers are shipped in a dry state. Even if shipped wet, or if cleaned for reuse using an aqueous cleaner or disinfectant, the dialyzer may be allowed to dry before CO₂ gas prime. It is also contemplated to pump heated air from dialysis fluid circuit 30 to blood circuit 100 if needed to aid in such drying. Also, to the extent that blood pump 112, e.g., a peristaltic blood pump, occludes arterial line 104, a nurse or clinician may be instructed to leave the arterial line 104 disconnected from the actuator of blood pump 112 during the CO₂ gas prime. If arterial line 104 is placed in operable communication with blood pump 112, however, it is desirable to run the blood pump during the CO₂ gas priming so that air is more readily removed from arterial line 104 and so that CO₂ gas reaches all portions of blood set 100.

A priming fluid, such as dialysis fluid formulated for treatment or saline, is then used to remove CO₂ gas from blood circuit 100. Dialysis fluid formulated for treatment is delivered in one backfiltration priming embodiment from dialysis fluid circuit 30, e.g., via fresh dialysis fluid line 76 to blood circuit 100 via dialyzer 102, while saline may alternatively be delivered directly to arterial line 104 or venous line 106, e.g., via blood pump 112 or perhaps via gravity. It is contemplated that the priming will displace the CO₂ gas in two ways. In one way, the heavier priming fluid pushes the CO₂ gas from hydrophobic or hydrophilic vent 116 located on venous drip chamber 114 just like the CO₂ gas pushed the lighter air from vent 116. In another way, the dialysis fluid or saline under higher pressure, e.g., while running blood pump 112, will absorb or dissolve the CO₂ gas.

It is contemplated that the purging in combination with the absorbing or dissolving of the CO₂ gas will remove most all of the gas prior to treatment. It should be appreciated however that CO₂ is a physiologically compatible substance that is readily absorbed in the patient's bloodstream and removed from the body via breathing. Thus trace amounts of CO₂ remaining after the fluid prime are not harmful to the patient and are instead handled naturally. It is believed that the two stage, CO₂ gas and fluid prime, even further improves air removal.

In addition to blood set or circuit 100, control unit 20 may also be programmed to remove any remaining CO₂ priming fluid including its constituents from gas generation chamber 90 prior to allowing the patient to connect to blood set 100 and/or prior to performing a subsequent CO₂ priming sequence. In one embodiment, control unit 20 causes metering pump 82 to run in reverse, pulling any remaining fluid from gas generation chamber 90 through three-way solenoid valve 192b, and pushing the fluid back through three-way solenoid valve 192a, priming fluid line 136 and B-concentrate line 36 into fresh dialysis fluid line 76, and via fluid bypass line 74 into drain line 56 to drain 60. CO₂ gas from CO₂ gas line 86 backfills the fluids removed from gas generation chamber 90. Gas generation chamber 90 may therefore be filled with atmospheric CO₂ gas upon commencing a subsequent CO₂ priming sequence. Care should be taken that only CO₂ gas, and no fluid from dialyzer 102, is pulled into gas generation chamber 90 to avoid any potential contamination. In this sequence, concentrate pumps 38 and 44 may be locked at their present flow rates. Conductivity cell 46 may be used with control unit 20 at the end of the sequence to confirm fluid removal from gas generation chamber 90.

The above example described the preparation of CO₂ gas using a separate citric acid supply 28. In an alternative embodiment, citric acid supply 28 and associated citric acid line 128 are removed, and an A-concentrate from A-concentrate source 24 is used instead. A-concentrates vary in physiological composition from manufacturer to manufacturer and from type to type. It is believed however that most if not all A-concentrates will work at least to some degree in combination with bicarbonate to create CO₂. One suitable A-concentrate for preparing CO₂ gas has a dilution ratio if 45 (e.g., having 135 mmol/l of acetic acid).

With the removal of citric acid supply 28 and associated citric acid line 128, A-concentrate may be pumped from A-concentrate source 24, through a portion of A-concentrate line 34 and A-concentrate priming line 134, to the same port of three-way solenoid valve 192a that connected formerly to citric acid line 128. In the illustrated arrangement of Fig. 1A, A-concentrate may therefore be metered via metering pump 82 to gas generation chamber 90 in the same manner described above for citric acid.

Fig. 1A illustrates an either/or scenario in which either (i) citric acid supply 28 and associated citric acid line 128 are present while priming line 134 is not present, or (ii) A-concentrate priming line 134 is present to use A-concentrate from A-concentrate source 24 instead, while citric acid supply 28 and associated citric acid line 128 are not present. It is expressly contemplated however for system 10b to provide both options at once, in which both citric acid supply 28 and associated citric acid line 128 and A-concentrate priming line 134 are provided. Here, a three-way valve under control of control unit 20, e.g., similar to three-way solenoid valves 192a and 192b, may be added at the juncture of lines 28 and 134 to allow control unit 20 to open either a path including citric acid supply 28 and associated citric acid line 128 or a path including A-concentrate priming line 134 and A-concentrate source 24, as desired by the operator.

Like above, with the fluid pathway through three-way solenoid valve 192b to metering pump 82 opened, and valve 92 in CO₂ gas line 86 open or closed as desired, the A-concentrate and bicarbonate solution may be allowed to mix for a specified period of time, e.g., for enough time to allow a downstream underpressure to be formed to pull CO₂ gas out of the mixture, producing a volume of CO₂ gas that will pressurize inside gas generation chamber 90. Once the specified CO₂ gas production period has ended, and with the most downstream valve 92 in fresh dialysis fluid line 76 closed and the fluid pathway through second three-way solenoid valve 192b to metering pump 82 opened to allow for circulation within the loop including lines 84 and 136, control unit 20 causes valve 92 in CO₂ gas line 86 to open (if previously closed), allowing the pressurized CO₂ gas inside chamber 90 to flow through CO₂ gas line 86, a distal portion of dialysis fluid line 76, and fresh dialysis fluid tube 78 into blood circuit 100 via dialyzer 102. If valve 92 in CO₂ gas line 86 has instead been open during CO₂ gas formation, CO₂ gas migrates naturally along CO₂ gas line 86 as discussed above. Blood circuit 100 is then primed with CO₂ gas as described above.

It should be noted that when using A-concentrate to create carbon dioxide gas, it is likely that an underpressure needs to be created downstream from gas generation chamber 90 for the CO₂ to be released from the mixed solutions. For example, with dialyzer 102 being dry, control unit 20 may cause blood pump 112 to run in reverse to pull a negative pressure through arterial line 104 leading to dialyzer 102, the dialyzer itself, fresh dialysis fluid tube 78 leading to dialyzer 102, CO₂ gas line 86, and a distal portion of dialysis fluid line 76. Also, system 10a using A-concentrate to create carbon dioxide gas is configured to mitigate or prevent the creation of precipitation, such as calcium carbonate. With A-concentrate, the amount (or perhaps a concentration of the concentrate leading to the amount) of acid in the solution is selected to create an amount of CO2 gas needed for priming.

It is also contemplated that when using A-concentrate for the acid solution, a surplus of the A-concentrate may be needed. Without the surplus, it is more likely that precipitation will form in gas generation chamber 90, which is undesirable. Assuming the use of a bicarbonate concentrate having a concentration of 1000 mmol/l (e.g., from B-concentrate in liquid form stored in a canister), pumping 10 mL (see table below) of that B-concentrate into the chamber will produce 10 mmol of sodium bicarbonate. In comparison, pumping 85 ml of 45x A-concentrate (as is done in the table below) will produce about 11.475 mmol of HAc. But if instead BiCart^{®} powdered bicarbonate cartridge solution provided by the assignee of the present disclosure is used, the concentration of 10mL of resulting bicarbonate fluid leaving the cartridge is in the range of 1200 mmol/l depending upon temperature. Here, to minimize precipitation, approximately 1200/1000 (20% more) 45x A-concentrate may be added, resulting in about 100 mL of A-concentrate and 13.5 mmol HAc. 100 mL of A-concentrate and 10 mL of BiCart^{®} produced bicarbonate solution will then yield about 330 to 350 mL of CO₂ gas depending on the temperature of the mixture. To produce instead a desired lesser amount of CO₂ gas (e.g., 250 mL), the amount of sodium bicarbonate is lowered to in turn lower the A-concentrate needed.

In addition to citric acid and A-concentrate, other acidic solutions may be combined with the bicarbonate solution to produce CO₂ gas. One such acidic solution is marketed under the tradename SelectBag^{®}, which is an acetic acid containing concentrate developed by the assignee of the present disclosure. The SelectBag^{®} solution may be placed at the location of either citric acid bag 28 or A-concentrate container 24 and used according to the corresponding sequence described above. 20 mL of SelectBag^{®} solution (example used in the table below) contains approximately 12 mmol HAc. The amount of sodium bicarbonate should be in the same range to avoid precipitation using SelectBag^{®} solution.

Citric acid from source 28 includes no calcium chloride in one embodiment and therefore does not yield a precipitate. Alternatively, when using a dialysis concentrate that contains calcium chloride, for example, system 10a ensures that enough concentrate is provided, so that the pH is held low enough to prevent the formation of precipitation. The SelectBag^{®} solution, for example, contains calcium and acetic acid. When using SelectBag^{®} solution, system 10a again ensures that enough concentrate is added to prevent precipitation due to the calcium. Another possible solution is SelectBag^{®} Citrate solution provided by the assignee of the present disclosure. SelectBag^{®} Citrate solution contains calcium and citric acid. When using SelectBag^{®} Citrate solution, system 10a yet again ensures that enough concentrate is added to prevent precipitation, despite the fact that the solution contains citric acid (even though the citric acid improves the situation because citric acid binds with calcium, lowering the concentration of free ionized calcium, and reducing the risk for precipitation). It is only when a precipitation yielding component such as calcium chloride is not present, such as with pure citric acid, that precipitation is not possible. Even here however, system 10a provides enough acid to yield the correct or desired amount of CO₂ gas.

Other suitable acid solutions or blends thereof include a lactic acid solution, a malic acid solution, a hydrochloric ("HCl") acid solution, (vi) a phosphoric acid solution, and (vii) any other biocompatible acid solution. Any one or more of the above-listed acid solutions may be mixed or blended with each other as desired for use with system 10a (and any of the systems described herein). It should also be appreciated that while the mixing of bicarbonate solution and an acid solution has been described as delivering bicarbonate solution first, then acid solution, the order may be reversed for any of the embodiments described herein.

As discussed above, it is contemplated to create and deliver a volume of CO₂ gas that is a multiple of the total volume of blood set 100 to ensure proper removal of air. The following table shows experimental data performed using saturated B-concentrate mixed alternatively with 45x A-concentrate (mix one part of the concentrate and 44 parts purified water to make 45 parts of ready fluid), SelectBag^{®} solution, and 50% by volume citric acid. Assuming blood set 100 to be 250 mL in total volume, the liquid volumes specified in the table would need to be increased three to seven times to achieve sufficient multiples of the blood set volume. The use of citric acid for example would require 50 to 60 mL of total liquid volume to achieve a 4X multiplier of the blood set volume. 60 mL of total liquid volume is believed to be a very reasonable amount.

| vol. sat. B conc. liquid mL | vol. 45x A conc. liquid mL | vol. SelectBag liquid mL | vol. 50% citric acid liquid mL | vol. CO₂ gas mL |
|---|---|---|---|---|
| 10 | 85 | | | 290 |
| 10 | | 10 | | 150 |
| 10 | | 20 | | 230 |
| 10 | | | 2 | 250 |
| 10 | | | 2 | 210 |

Please note that the above data is for mixing at atmospheric pressure and a temperature of around 30°C to 35°C. It should also be understood that the numbers above are illustrated for the purpose of showing that enough CO₂ gas may be produced using a reasonable amount of CO₂ gas generation fluid constituents. The actual amounts used in practice will likely be adjusted when precipitation is taken into account. Moreover, reducing downstream pressure, e.g., by reducing the pressure in arterial line 104, reduces the amount of CO₂ gas needed (e.g., half the quantity needed at half atmospheric pressure in the bloodlines). It is accordingly contemplated to maintain a low pressure in blood set 100, e.g., at atmospheric or below, during the CO₂ gas priming.

Moreover, the example above using 10 mL^{®} solution will likely create significant precipitation and thus may not present a realistic solution. The two citric acid examples having two different input volumes yielding different gas volumes show that test results may vary, for example, based upon environmental temperature and whether or not an underpressure is provided with the reaction. The underpressure may yield gas volume results that exceed what is theoretically possible at atmospheric pressure.

The formulations for the CO₂ gas priming mixtures are very likely going to be different than the formulations for treatment fluid, either because different constituents are used or because the same constituents are used in different proportions. Regarding the formulations generally, mixtures may be prepared accurately in two different ways, either by (i) using a formula for the mixture and accurately adding the constituents according to the formula or (ii) using a sensor reading indicative of the overall constituency of the mixture, e.g., conductivity, and sensor feedback to control unit 20 to servo to a desired sensor reading setpoint for a desired overall constituency. The different CO₂ gas formulations of the present disclosure may be prepared using either mixing accuracy technique.

The above table shows formulas, e.g., (a) formulas 1:8.5 for bicarbonate/A concentrate, (b) formulas 1:1 and 1:2 for bicarbonate/SelectBag^{®} solution, and (c) formula 5:1 for bicarbonate/citric acid. As discussed above, it is conceivable to use conductivity sensors, setpoints and servo feedback to meet setpoints instead of the above formulas for the priming fluid mixtures. But in any case, the formulation of CO₂ gas generation fluid will be different in at least one respect than the formulation of fresh dialysis fluid prepared for treatment.

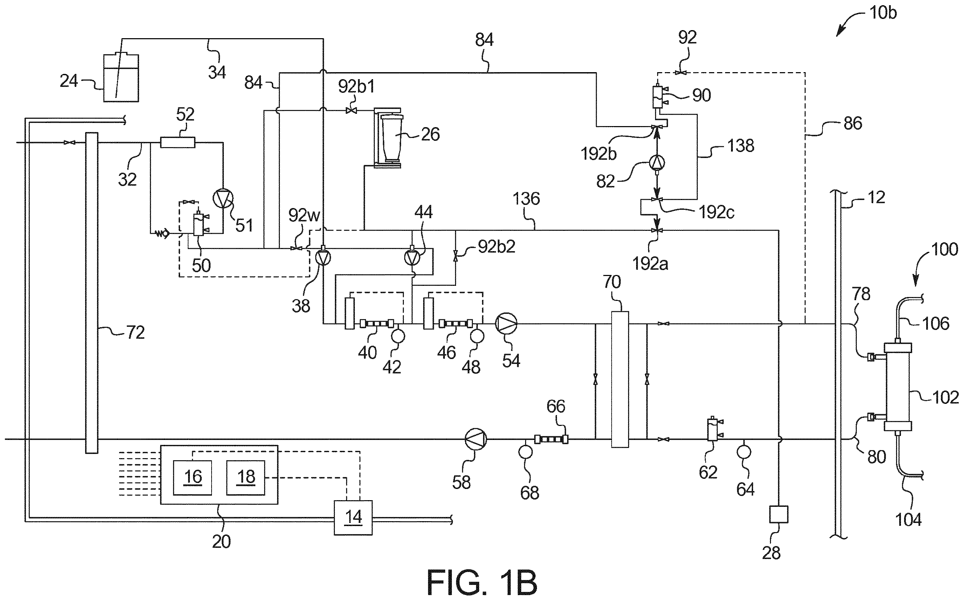

Referring now to Fig. 1B an alternative system for making CO₂ gas in dialysis fluid circuit 30 and delivering such gas to blood circuit 100 is illustrated by system 10b. System 10b is the same in many respect to system 10a (including all structure, functionality and alternatives discussed above). System 10b adds a recirculation line 138 extending in the illustrated embodiment from a bottom portion of gas generation chamber 90 back to a third three-way valve 192c under control of control unit 20, and located upstream of metering pump 82. Bicarbonate and acid solutions are delivered to gas generation chamber 90 as described above, but here with the path through third three-way valve 192c to recirculation line 138 closed. Once the desired amount of bicarbonate and acid solutions are delivered to gas generation chamber 90, the state of third three-way valve 192c is switched such that the path through third three-way valve 192c to three-way valve 192a is closed, while the path through third three-way valve 192c to recirculation line 138 is opened.

During and after formation of the CO₂ gas, metering pump 82 recirculates the CO₂ gas generation fluid down recirculation line 138 and back up through third three-way valve 192c to gas generation chamber 90. The recirculation stirs and agitates the CO₂ gas generation fluid, causing CO₂ gas to be released from the fluid and to flow out of gas generation chamber 90 through gas line 86. In an embodiment, recirculation line 138 may be fitted with a spiraled member, a staggered flanged member, or other type of turbulator to further disrupt and turbulate the CO₂ gas generation fluid and release the CO₂ gas.

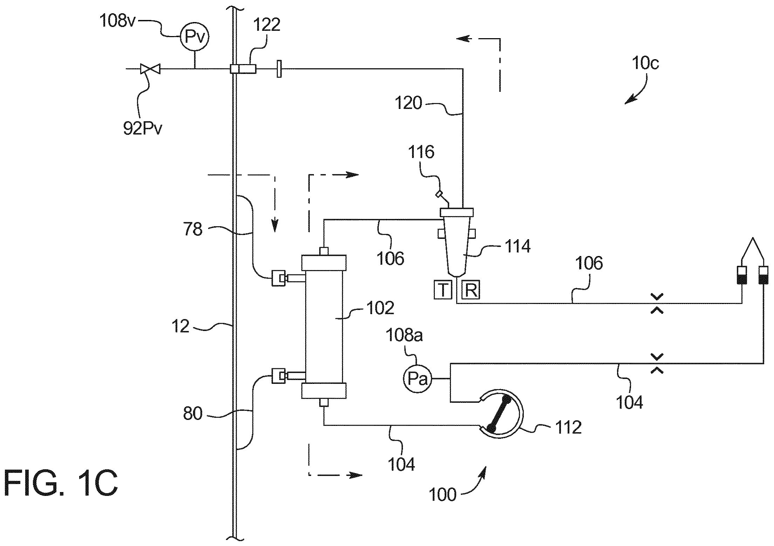

Referring now to Fig. 1C, an alternative system for delivering CO₂ gas from dialysis fluid circuit 30 to blood circuit 100 is illustrated by system 10c. System 10c is the same in many respect to system 10a (including all structure, functionality and alternatives discussed above). System 10c may also include recirculation line 138, third three-way valve 192c, and the associated structure and functionality discussed in connection with system 10b. System 10c adds a pressure measurement line 120 leading from a top of drip chamber 114 to a machine connector 122. In the illustrated embodiment, CO₂ gas is produced within machine 12 and is delivered to blood set 100 as described above. Control unit 20 causes blood pump 112 to run slowly to remove air from arterial line 104 and venous line 106 (connected together in Fig. 1C) and to deliver the air into venous drip chamber 114. Air in chamber 114 is in turn led out of blood set 100 via venous pressure line 120 into the interior of machine 12 via valve 92Pv under control of control unit 20.

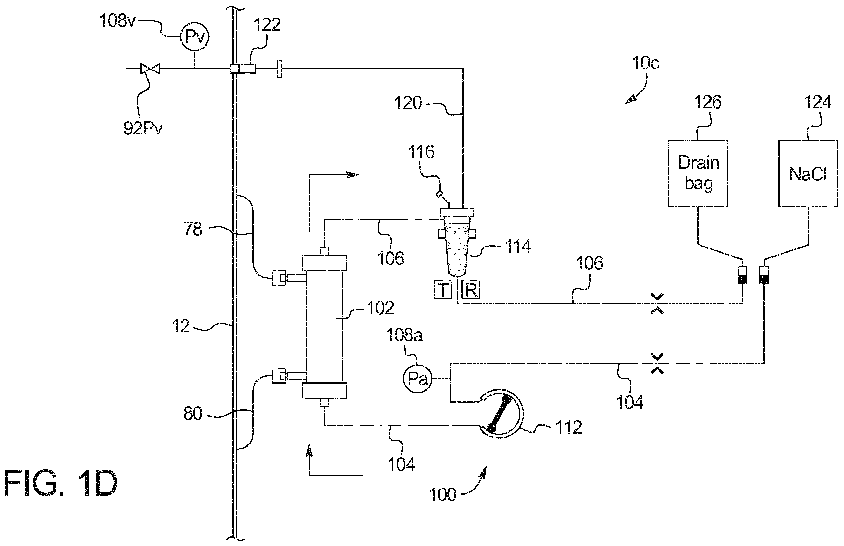

Referring now to Fig. 1D, one embodiment for a priming fluid portion of the priming of blood circuit 100 of system 10c is illustrated. Here, the patient ends of arterial line 104 and venous line 106 are disconnected from each other (connected in Fig. 1C). After CO₂ gas priming has been completed, a saline bag or container 124 is connected to the distal end of arterial line 104, while an empty drain bag or container 126 is connected to the distal end of venous line 106. Blood pump 112 under control of control unit 20 is operated to pull saline from saline bag or container 124 through the portion of arterial line 104 upstream of blood pump 112, and to push saline through the portion of arterial line 104 downstream from blood pump 112, dialyzer 102, drip chamber 114 and venous line 106 to drain bag or container 126. The saline prime pushes CO₂ gas both to drain bag or container 126 and through venous pressure line 120 into the interior of machine 12 via valve 92Pv under control of control unit 20 until a level detector operable with drip chamber 114 detects a high fluid level, after which valve 92Pv is closed. Additionally, as discussed above, saline especially under positive pressure will also absorb some of the CO₂ gas back into solution. Moreover as discussed above, small amounts of CO₂ gas are not harmful to the patient.

In each of the previous examples, CO₂ gas is created in dialysis fluid circuit 30 and delivered in gaseous form to blood circuit 100. In an alternative primary embodiment illustrated in Figs. 2A to 2D, system 110a instead causes CO₂ gas to be carried within the formulated priming fluid into blood circuit 100, where the CO₂ gas is then degassed from the priming fluid to help rid blood circuit 100 of small air pockets. System 110a may include any of the structure, functionality and alternatives discussed above for systems 10a, 10b and 10c in combination with the additional structure, functionality and alternatives described below for Figs. 2A to 2D.

In Figs. 2A to 2D, blood circuit 100 operates with dialysis fluid circuit 30 as shown above, except that gas generation chamber 90 and associated plumbing is not needed or provided. Figs. 2A to 2D also illustrate that machine 12 may further include a hemodialfiltration ("HDF") port 94 and/or a waste handling option ("WHO") port 98. As mentioned above, a portion of fresh dialysis fluid flowing along fresh dialysis fluid line 76 may be diverted through one or more ultrafilter to purify the fresh dialysis fluid to such a level that it may be delivered as replacement fluid directly into arterial line 104 as illustrated to perform pre-dialyzer or pre-infusion HDF or into venous line 106 to perform post-dialyzer or post-infusion HDF. Figs. 2A to 2D illustrate a replacement fluid pump 96, which under control of control unit 20 delivers the replacement fluid to blood circuit 100 at a desired flowrate. Figs. 2A to 2D illustrate that arterial line 104 and venous line 106 may be plugged together into WHO port 98 to form a loop for the priming fluid and CO₂ gas.

It should be appreciated that in any embodiment described herein, CO₂ gas, e.g., via gas generation chamber 90 of system 10a, or via priming fluid in system 110a, may be introduced into blood circuit 100 (i) via fresh dialysis fluid line 76 and tube 78 or (ii) via a pre-, post-, or pre- and post- HDF port 94. In a further alternative embodiment, CO₂ gas may be introduced into blood circuit 100 via an air vent line running between drip chamber 114 and hydrophobic or hydrophilic vent 116.

In Fig. 2A, control unit 20 of system 110a causes replacement fluid pump 96 to pump priming fluid formulated to create CO₂ gas into blood circuit 100. The priming fluid formulated to create CO₂ may be created using bicarbonate solution and any of the acid solutions described above, e.g., (i) a citric acid solution, (ii) an acetic acid solution (e.g., from A-concentrate), (iii) a lactic acid solution, (iv) a malic acid solution, (v) a hydrochloric ("HCl") acid solution, (vi) a phosphoric acid solution, (vii) any other biocompatible acid solution, and (v) blends thereof. The flowrate of priming fluid formulated to create CO₂ gas may be a desired flowrate Qₚ mL/minute (e.g., from 100 to 500 mL/minute) at replacement fluid pump 96 and below Qₚ mL/minute at blood pump 112. The introduction of the priming fluid into blood circuit 100 causes much of the air to be pushed out of blood circuit 100 via hydrophobic or hydrophilic vent 116.

In Fig. 2B, control unit 20 causes the speed of blood pump 112 to be increased, e.g., above Qₚ mL/minute, so that blood pump 112 pumps faster than replacement fluid pump 96, which creates a relatively lower pressure in replacement fluid line 118. Blood pump 112 pumping towards dialyzer 102 also places venous line 106 and the portion of arterial line 104 upstream of blood pump 112 under negative pressure. Venous drip chamber 114 with a hydrophilic filter 116 is inverted, e.g., manually, or via an electromechanical actuator under control of control unit 20 in Fig. 2B, so that air is not pulled into blood circuit 100. The relatively low pressure in replacement fluid line 118 and the negative pressure in venous line 106 and in the portion of arterial line 104 upstream of blood pump 112 both cause the priming fluid to degas CO₂ into those portions of blood circuit 100. The CO₂ gas dislodges air pockets in tight portions of blood circuit 100 as has been described above.

Control unit 20 may then cause blood pump 112 in Fig. 2B to move in reverse to place the priming fluid in the remaining portion of arterial line 104 and dialyzer 102 under negative pressure to cause the priming fluid to degas CO₂ into the remainder of arterial line 104 and dialyzer 102. The CO₂ gas dislodges air pockets in the remainder of arterial line 104 and in the membranes and pores of the membranes of dialyzer 102 as has been described above. Control unit 20 may cause blood pump 112 to reverse back and forth multiple times to repeat the above-described sequence multiple times to ensure that blood circuit 100 has been subjected fully to CO₂ gas, so that virtually all air within blood circuit 100 has been dislodged for removal.

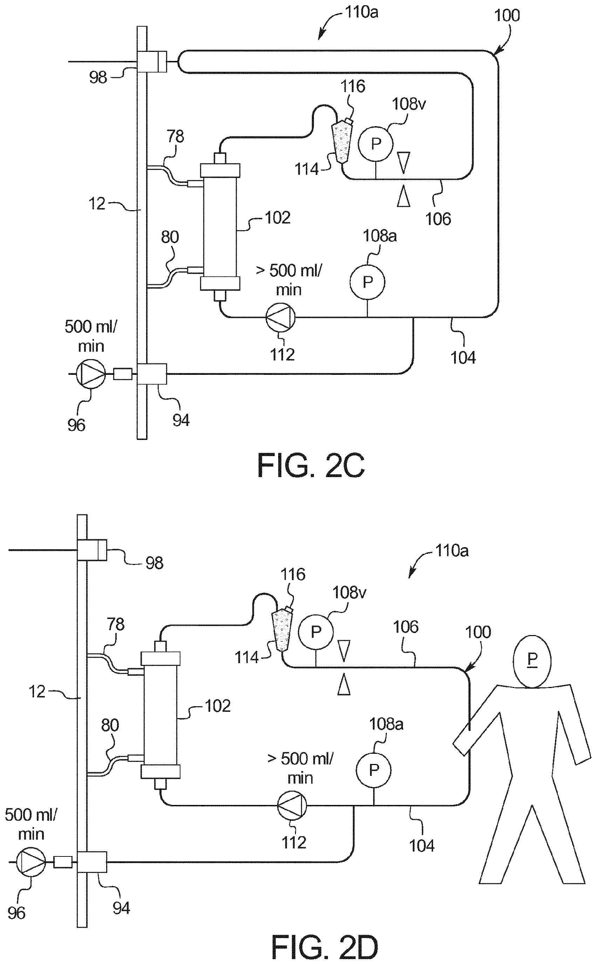

Fig. 2C illustrates that after the blood circuit 100 has been subjected fully to CO₂ gas, venous drip chamber 114 may be flipped to its normal position, e.g., manually, or via an electromechanical actuator under control of control unit 20. Control unit 20 may again reverse blood pump 112 multiple times to ensure that all parts of blood circuit 100 see positive pressure leading to venous drip chamber 114 and its vent 116, so that virtually all air within blood circuit 100 has been dislodged for removal.

Fig. 2D illustrates patient P connected to arterial line 104 and venous line 106 for treatment. Control unit 20 may be programmed such that after the CO₂ gas prime of Figs. 2A to 2C, CO₂ gas and any remaining CO₂ priming fluid for preparing the CO₂ gas may be absorbed and/or displaced using dialysis fluid prepared for treatment or saline. As discussed above, CO₂ is a physiologically compatible substance that is readily absorbed in the patient's bloodstream and removed from the body via breathing. Any trace amount of CO₂ remaining after the fluid prime of Figs. 2B and 2C is not harmful to the patient and is instead handled naturally.

Referring now to Figs. 3A to 3D, system 110b illustrates an alternative arrangement for carrying CO₂ gas within the priming fluid formulated to produce CO₂ gas into blood circuit 100, where the CO₂ gas is then degassed from the priming fluid to help rid blood circuit 100 of small air pockets. System 110b includes all structure, functionality and alternatives discussed above and incorporated into system 110a. System 110b moves replacement fluid line 118 and replacement fluid pump 96 as shown in the illustrated embodiment, so that replacement fluid is delivered via pump 96 under control of control unit 20 to venous line 106 (post-dilution) instead of to arterial line 104 (pre-dilution) in Figs. 2A to 2D. Either system 110a and 110b may be configured to have pre- or post- dilution or pre- and post-dilution replacement fluid delivery. System 110b also moves hydrophilic vent 116 to be just in front of, or to be part of, machine connector 122. Any of systems 10a to 10c, 110a or 110b may have hydrophilic vent 116 arranged as shown in Figs. 3A to 3D.

System 110b of Figs. 3A to 3D in the illustrated embodiment also includes a WHO pump 130 located within machine 12 (WHO pump may be any of the types of pumps described for metering pump 82), which is under control of control unit 20 and is positioned and arranged to pull liquid from blood set 100 via WHO port 98. A WHO valve 92_{WHO} under control of control unit 20 is located within machine 12 between WHO port 98 and WHO pump 130. Any of systems 10a to 10c, 110a or 110b may have WHO pump 130 and WHO valve 92_{WHO} arranged as shown in Figs. 3A to 3D. System 110b of Figs. 3A to 3D in the illustrated embodiment further includes an air pump 132 located within machine 12 (air pump 132 may be any of the types of pumps described for metering pump 82), which is under control of control unit 20 and is positioned and arranged to push or pull air to or from venous drip chamber 114 of blood set 100. Air pump 132 is located between machine connector 122 and valve 92Pv in the illustrated embodiment. Any of systems 10a to 10c, 110a or 110b may have air pump 132 arranged as shown in Figs. 3A to 3D.

System 110b of Figs. 3A to 3D further includes a level sensor 142 operable with venous drip chamber 114 and located to sense a level of liquid within the chamber. Level sensor 142 sends a signal indicative of the level of liquid within chamber 114 to control unit 20, which reacts accordingly. Any of systems 10a to 10c, 110a or 110b may have a level sensor 142 arranged as shown in Figs. 3A to 3D. System 110b of Figs. 3A to 3D also includes a replacement fluid line clamp 140 under control of control unit 20. Replacement fluid line clamp 140 allows or disallows replacement fluid and other fluids to flow to venous line 106 of blood set 100. Any of systems 10a to 10c, 110a or 110b may have a replacement fluid line clamp 140 arranged as shown in Figs. 3A to 3D. Still further, System 110b of Figs 3A to 3D may include a gas/liquid sensor 144 under control of control unit 20. Gas/liquid sensor 144 is configured to sense the difference between CO₂ gas generation fluid and CO₂ gas or air residing just below venous drip chamber 114. Any of systems 10a to 10c, 110a or 110c may have a gas/liquid sensor 144 arranged as shown in Figs. 3A to 3D.

In Fig. 3A system 110b fills venous drip chamber 114 with CO₂ gas generation fluid. In one embodiment, control unit 20 causes replacement fluid pump 96 to run and fill venous drip chamber 114. WHO pump 130 does not need to be operated. Here, in one embodiment, CO₂ gas generation fluid and any escaping CO₂ gas force air out of blood set 100 through drip chamber 114, via venous pressure line 120 and into the interior of machine 12 via valve 92Pv under control of control unit 20.

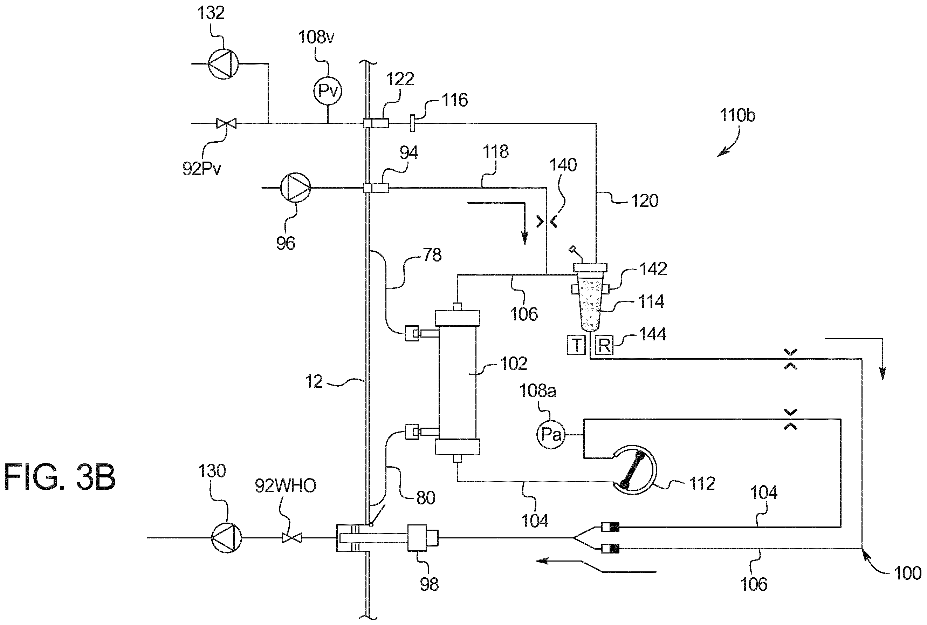

In Fig. 3B, when level sensor 142 at venous drip chamber 114 senses a high level of CO₂ gas generation fluid, CO₂ gas generation fluid is pumped or gravity flowed out the bottom of venous drip chamber 114 towards WHO port 98 via venous line 106. Control unit 20 may be programmed to allow CO₂ gas generation fluid to flow out the bottom of venous drip chamber 114 for a preset period of time or until receiving a signal from a sensor, such as an ultrasonic or capacitive sensor (not illustrated), positioned at a desired location upstream of WHO port 98 to detect that no more air is coming down the line.

Control unit 20 then causes blood pump 112 to pull CO₂ gas generation fluid through connected venous line 106 and arterial line 104 to fill arterial line 104 up to dialyzer 102.

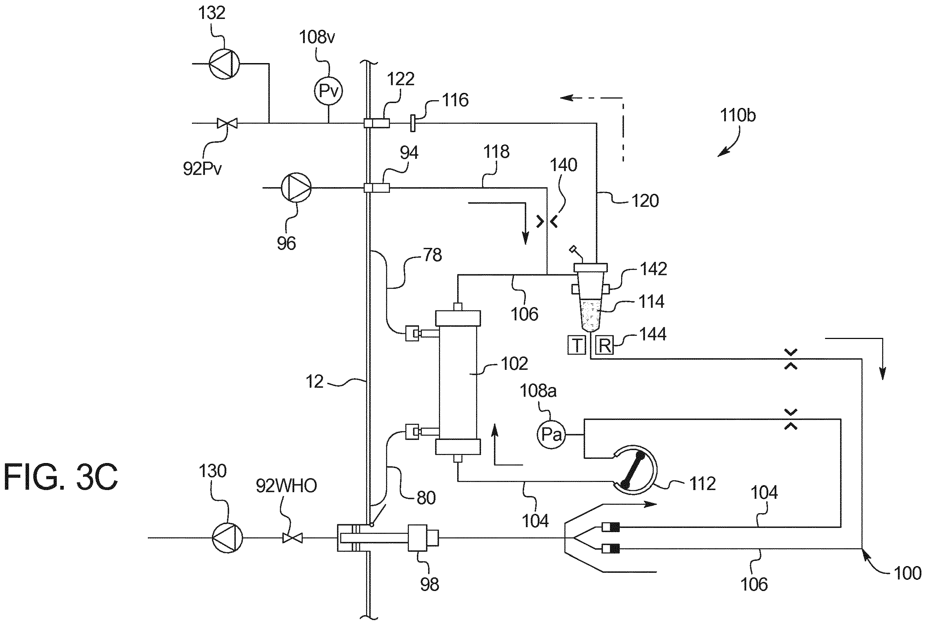

Fig. 3C illustrates one embodiment for filling arterial line 104 between blood pump 112 and dialyzer 102, the dialyzer itself, and the remainder of venous line 106 with CO₂ gas generation fluid. Control unit 20 causes blood pump 112 to pump CO₂ gas generation fluid somewhat faster than the rate at which replacement fluid pump 96 introduces new CO₂ gas generation fluid into blood circuit 100, so that blood pump 112, with arterial and venous lines 104 and 106 connected together as illustrated and with WHO valve 92_{WHO} closed, pulls CO₂ gas generation fluid out of venous drip chamber 114 more quickly than replacement fluid pump 96 refills the chamber, thereby lowering the liquid level in venous drip chamber 114. The output of blood pump 112 fills the positive pressure portion of arterial line 104, dialyzer 102 and the portion of venous line 106 up to the inlet of replacement fluid line 118 with CO2 gas generation fluid. Air is again forced out of blood set 100 through drip chamber 114, via venous pressure line 120 and into the interior of machine 12 via valve 92Pv under control of control unit 20. When all lines of blood set 100 are filled with CO₂ gas generation fluid, the liquid level within venous drip chamber 114 will rise, eventually tripping level sensor 142, telling control unit 20 that blood set 100 is full.

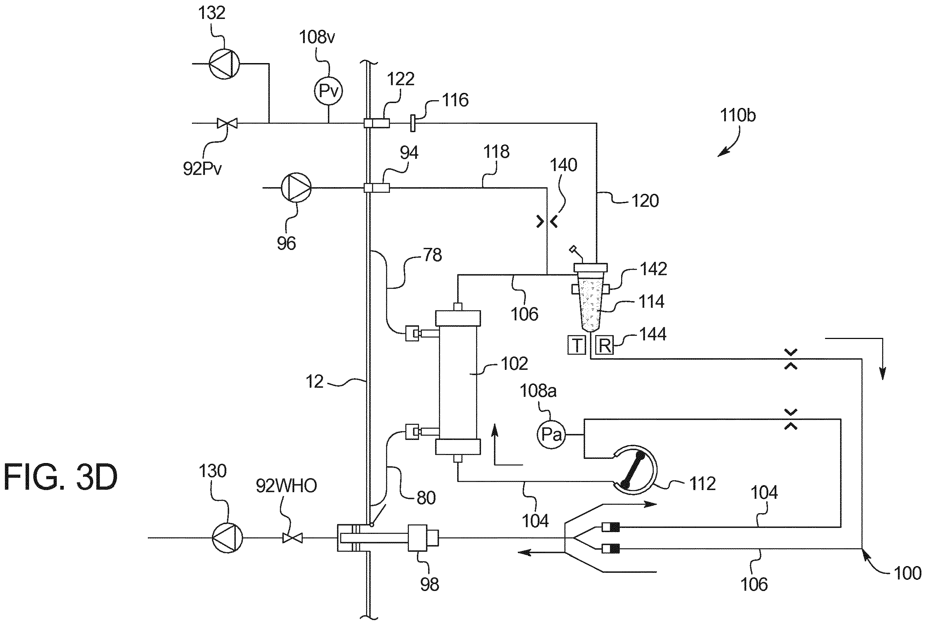

Fig. 3D illustrates one embodiment for creating a low pressure in blood set 100 to pull CO₂ gas out of the CO₂ gas generation fluid. Here, control unit 20 causes replacement fluid pump 96 to stop and closes replacement fluid line clamp 140. Control unit 20 further causes (i) WHO pump 130 to create a suction through WHO valve 92_{WHO} to the connection of arterial line 104 and venous line 106, and (ii) blood pump 112 to run slowly to remove gas created inside of dialyzer 102 due to the degassing and to deliver CO₂ gas to all areas of blood set 100. The suction pressure releases CO₂ gas from CO₂ gas generation fluid as has been discussed herein. If the level of CO₂ gas generation fluid in venous chamber 114 falls too low, control unit 20 may open replacement fluid line clamp 140 and cause replacement fluid pump 96 to restore the CO₂ gas generation fluid level.

When CO₂ gas priming is completed, a saline prime may be performed as illustrated in connection with Fig. 1D, or dialysis fluid for treatment may be used to flush CO₂ gas through drip chamber 114, via venous pressure line 120 and into the interior of machine 12 via valve 92Pv under control of control unit 20. CO₂ gas may additionally be absorbed into saline or dialysis fluid for treatment as described herein.

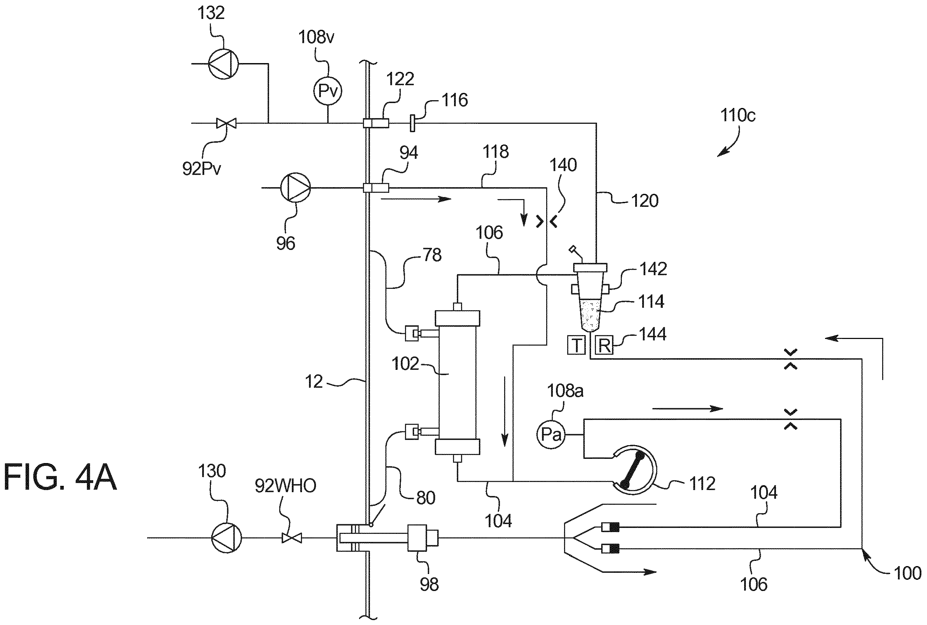

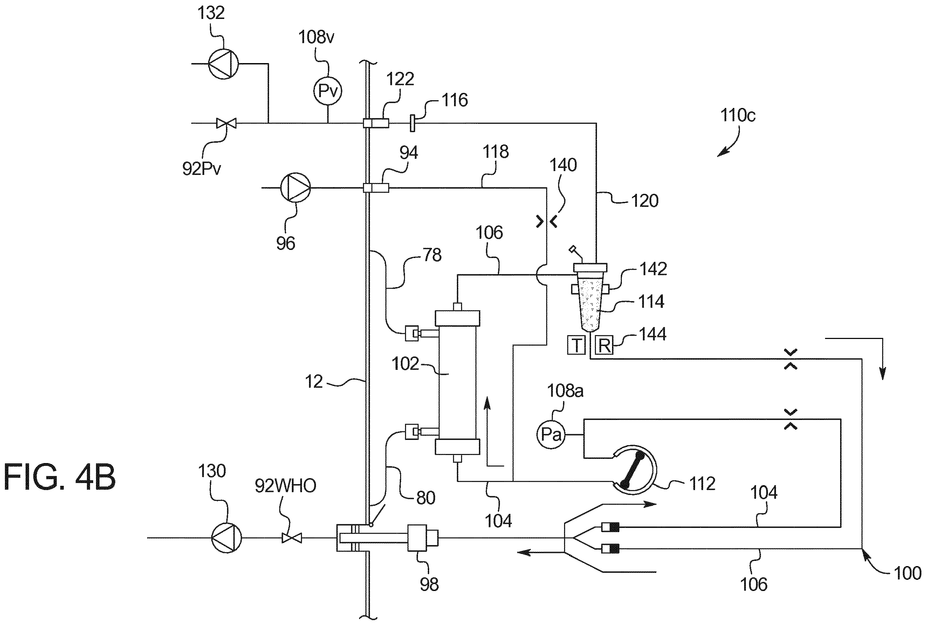

Referring now to Figs. 4A and 4B, system 110c illustrates an alternative arrangement for carrying CO₂ gas within the priming fluid formulated to produce CO₂ gas into blood circuit 100, where the CO₂ gas is then degassed from the priming fluid to help rid blood circuit 100 of small air pockets. System 110c includes all structure, functionality and alternatives discussed above and incorporated into systems 110a and 110b. System 110c moves replacement fluid line 118 as shown in the illustrated embodiment, so that replacement fluid is delivered via replacement fluid pump 96 under control of control unit 20 to arterial line 104 (pre-dilution) as with in Figs. 2A to 2D.

Pre-dilution system 110c, unlike pre-dilution system 110a in Figs. 2A to 2D, uses valve 92Pv inside machine 12 to close off the air path when needed. It is noted that filter 116 in system 110c is a hydrophilic filter in the illustrated embodiment. Filter 116 may be used to protect blood from entering and contacting the connection site at machine connector 122 and the inside of machine 12.

Fig. 4A illustrates one example embodiment for filling blood set 100. Control unit 20 with replacement fluid line clamp 140 open causes blood pump 112 to run in reverse direction at a desired speed, pushing CO₂ gas generation fluid through connected lines 104 and 106 and backwards through venous line 106 to venous drip chamber 114. Fluid/gas sensor 144 in communication with control unit 20 eventually sees the CO₂ gas generation fluid, which control unit 20 reads and causes blood pump 112 to stop. Control unit 20 then causes replacement fluid pump 96 to pump CO₂ gas generation fluid to blood set 100 including dialyzer 102 and the portion of venous line 106 to drip chamber 114 until level detector 142 operable with drip chamber 114 sees a high level of fluid.

In Fig. 4B, system 110c with blood set 100 having been filled with CO₂ gas generation fluid and with replacement fluid line clamp 140 closed, may then create a low pressure in blood set 100 in the manner described above in Fig. 3D, releasing the CO₂ gas from the CO₂ gas generation fluid to perform the CO₂ gas priming as described herein. After the CO₂ gas prime, blood set 100 may then be primed with dialysis fluid for treatment as has been described herein.

element number listing
10a, 10b, 10c - systems
12 - machine
14 - user interface 14
16 - processor
18 - memory
20 - control unit
22 - purified water source
24 - A-concentrate source
26 - B-concentrate source or bicarbonate cartridge
28 - citric acid source or source of acid solution
30 - dialysis fluid circuit
32 - purified water line
34 - A-concentrate line
36 - B-concentrate line
38 - A-concentrate pump
40 - conductivity cell
42 - temperature sensor
44 - B-concentrate pump
46 - conductivity cell
48 - temperature sensor
50 - degassing chamber
51 - degassing pump
52 - heater
54 - fresh dialysis fluid pump
56 - used dialysis fluid line
58 - used dialysis fluid pump
60 - drain
62 - air separator
64 - pressure sensor
66 - conductivity cell
68 - temperature sensor
70 - UF system
72 - heat exchanger
74 - fluid bypass line
76 - fresh dialysis fluid line
78 - fresh dialysis fluid tube
80 - used dialysis fluid tube
82 - metering pump
84 - return line
86 - CO₂ gas line
90 - gas generation chamber or CO₂ gas chamber
92 - plural valves
92b1 and 92b2 - priming sequence valves
92Pv - air path valve
92w - water line valve
92_{WHO} - waste handling option valve
94 - hemodialfiltration ("HDF") port 94
96 - replacement fluid pump
98 - waste handling option ("WHO") port
100 - blood set or blood circuit
102 - dialyzer or blood filter
104 - arterial line
106 - venous line
108a - arterial pressure pod
108v - venous pressure pod
110a, 110b, 110c - systems
112 - blood pump
114 - venous drip chamber
116 - hydrophilic vent
118 - replacement fluid line
120 - pressure measurement line
122 - machine connector
124 - saline bag or container
126 - empty drain bag or container
128 - citric acid line
130 - WHO pump
132 - air pump
134 - A-concentrate priming line
136 - bicarbonate priming line
138 - recirculation line
140 - replacement fluid line clamp
142 - level sensor
144 - gas/liquid sensor
192a - first three-way solenoid valve
192b - second three-way solenoid valve

It should be understood that various changes and modifications to the presently preferred embodiments described herein will be apparent to those skilled in the art.

## Claims

1. An extracorporeal therapy system (10a to 10c) comprising:
a dialysis fluid circuit (30) including dialysis fluid preparation structure (22, 24, 26, 32, 34, 36, 38, 40, 42, 44, 46, 48) configured to prepare a dialysis fluid for an extracorporeal therapy treatment;
a blood circuit (100) including a blood filter (102) for use during the extracorporeal therapy treatment;
a blood pump (112) operable to pump blood through the blood circuit (100) and blood filter (102); and
a control unit (20) operable with the dialysis fluid preparation structure (22, 24, 26, 32, 34, 36, 38, 40, 42, 44, 46, 48) and the blood pump (112),
wherein said extracorporeal therapy system is **characterized by** the fact that the control unit (20) is programmed to prepare a gas generation fluid different than the dialysis fluid for the extracorporeal therapy treatment, wherein the gas generation fluid generates carbon dioxide ("CO₂") gas, and wherein the CO₂ gas is used to prime the blood circuit (100) including the blood filter (102).

2. The extracorporeal therapy system (10a to 10c) according to Claim 1, wherein the control unit (20) is further programmed to absorb and/or flush the CO₂ gas from the blood circuit (100) using a priming fluid, such as the dialysis fluid for the extracorporeal therapy treatment or saline, thereby further priming the blood circuit (100).

3. The extracorporeal therapy system (10a to 10c) according to Claims 1 or 2,
wherein the CO₂ gas is generated in the dialysis fluid circuit (30) and delivered from the dialysis fluid circuit (30) to the blood circuit (100).

4. The extracorporeal therapy system (10a to 10c) according to claim 1, wherein the CO₂ gas is carried by the gas generation fluid into the blood circuit (100), and wherein the CO₂ gas and remaining fluid is thereafter absorbed within and/or removed from the blood circuit (100) using a priming fluid, such as the dialysis fluid for the extracorporeal therapy treatment or saline.

5. The extracorporeal therapy system (10a to 10c) according to Claim 4, wherein the system is configured to lower the pressure of the gas generation fluid carrying the CO₂ gas to cause the CO₂ gas to be released.

6. The extracorporeal therapy system (10a to 10c) according to any of the preceding claims, wherein the blood circuit (100) includes a vented chamber (114) enabling at least one of (i) the CO₂ gas to push air out of a vent (116) of the vented chamber (114) or (ii) a priming fluid, such as the dialysis fluid for the extracorporeal therapy treatment or saline, to push CO₂ gas out of the vent (116).

7. The extracorporeal therapy system (10a to 10c) according to any of the preceding claims, which includes a CO₂ gas chamber (90) in fluid communication with the dialysis fluid preparation structure (22, 24, 26, 32, 34, 36, 38, 40, 42, 44, 46, 48), the CO₂ gas chamber (90) sized and arranged to mix the CO₂ gas generating priming fluid, causing CO₂ gas to be generated.

8. The extracorporeal therapy system (10a to 10c) according to Claim 7, which includes a metering pump (82) positioned and arranged to pump bicarbonate and (i) a citric acid solution, (ii) an acetic acid solution, (iii) a lactic acid solution, (iv) a malic acid solution, (v) a hydrochloric ("HCl") acid solution, (vi) a phosphoric acid solution, (vii) any other biocompatible acid solution, or (v) blends thereof.

9. The extracorporeal therapy system (10a to 10c) according to Claim 7, which includes a CO₂ gas line (86) extending from the CO₂ gas chamber (90) to a location where the CO₂ gas may be introduced into the blood circuit (100),
and wherein the location includes a line in fluid communication with a port of the blood filter (102) or a line in fluid communication with a blood line (104, 106) extending from the blood filter (102).

10. The extracorporeal therapy system (10a to 10c) according to Claim 7, wherein the CO₂ gas chamber (90) is separate from the blood filter (102).

11. The extracorporeal therapy system (10a to 10c) according to any of the preceding claims, wherein at least one of (i) a replacement fluid pump (96) is used to move the gas generation fluid while priming the blood circuit (100) or (ii) the blood pump (112) is operated in at least one direction while priming the blood circuit (100) with CO₂ gas, and/or wherein the dialysis fluid and the gas generation fluid are formed from at least one common concentrate (26).

12. The extracorporeal therapy system (10a to 10c) according to Claim 7, which includes a recirculation line (138) in fluid communication with the CO₂ gas chamber (90) to agitate the gas generation fluid to help release the CO₂ gas.

13. A priming method for an extracorporeal therapy system comprising:
storing in a computer memory (18) a first formula or setpoint for preparing a dialysis fluid for an extracorporeal therapy treatment;
storing in the computer memory (18) a second, different formula or setpoint for preparing a gas generation fluid for carbon dioxide ("CO₂") gas priming;
providing fluid preparation structure (22, 24, 26, 32, 34, 36, 38, 40, 42, 44, 46, 48) to prepare the gas generation fluid according to the second formula or setpoint;
allowing CO₂ gas to be generated from the gas generation fluid; and
priming a blood circuit (100) with the CO₂ gas, the blood circuit (100) including a blood filter (102).

14. The priming method according to Claim 13, wherein priming the blood circuit (100) with the CO₂ gas includes enabling the CO₂ gas to enter the blood circuit (100),
and wherein allowing the CO₂ gas to be generated and priming the blood circuit (100) with the CO₂ gas includes enabling the CO₂ gas to be released from the gas generation fluid after entering the blood circuit (100).

15. The priming method according to any of Claims 13 or 14,
wherein the second, different formula or setpoint for preparing the gas generation fluid involves (i) a mixing ratio between bicarbonate and a solution containing citric acid, (ii) a mixing ratio between bicarbonate and a solution containing acetic acid, (iii) a mixing ratio between bicarbonate and a solution containing lactic acid, (iv) a mixing ratio between bicarbonate and a solution containing malic acid, (v) a mixing ratio between bicarbonate and a solution containing hydrochloric ("HCl") acid, (vi) a mixing ratio between bicarbonate and a solution containing phosphoric acid, (vii) a mixing ratio between bicarbonate and any other biocompatible acid solution, or (v) blends thereof, , and/or wherein the second, different formula or setpoint for preparing the gas generation fluid includes a different constituency or a different make-up of a same constituency as the dialysis fluid for the extracorporeal therapy treatment.

## Patentansprüche

1. Extrakorporales Therapiesystem (10a bis 10c), umfassend:
einen Dialysefluidkreislauf (30), der Dialysefluidzubereitungsstruktur (22, 24, 26, 32, 34, 36, 38, 40, 42, 44, 46, 48) einschließt, die ausgestaltet ist, um ein Dialysefluid für eine extrakorporale Therapiebehandlung zuzubereiten;
einen Blutkreislauf (100), der einen Blutfilter (102) zur Verwendung während der extrakorporalen Therapiebehandlung einschließt;
eine Blutpumpe (112), die betriebsfähig ist, um Blut durch den Blutkreislauf (100) und den Blutfilter (102) zu pumpen; und
eine Steuereinheit (20), die betriebsfähig mit der Dialysefluidzubereitungsstruktur (22, 24, 26, 32, 34, 36, 38, 40, 42, 44, 46, 48) und der Blutpumpe (112) ist, wobei das extrakorporale Therapiesystem durch die Tatsache gekennzeichnet ist, dass die Steuereinheit (20) programmiert ist, um ein Gaserzeugungsfluid zuzubereiten, das sich von dem Dialysefluid für die extrakorporale Therapiebehandlung unterscheidet, wobei das Gaserzeugungsfluid Kohlendioxid ("CO₂")-Gas erzeugt, und wobei das CO₂-Gas zum Vorfüllen des Blutkreislaufs (100) einschließlich des Blutfilters (102) verwendet wird.

2. Extrakorporales Therapiesystem (10a bis 10c) nach Anspruch 1, wobei die Steuereinheit (20) des Weiteren programmiert ist, um das CO₂-Gas aus dem Blutkreislauf (100) unter Verwendung eines Vorfüllfluids, wie des Dialysefluids für die extrakorporale Therapiebehandlung oder Salzlösung, zu absorbieren und/oder auszuspülen, wodurch der Blutkreislauf (100) weiter vorgefüllt wird.

3. Extrakorporales Therapiesystem (10a bis 10c) nach Anspruch 1 oder 2, wobei das CO₂-Gas in dem Dialysefluidkreislauf (30) generiert und von dem Dialysefluidkreislauf (30) an den Blutkreislauf (100) abgegeben wird.

4. Extrakorporales Therapiesystem (10a bis 10c) nach Anspruch 1, wobei das CO₂-Gas durch das Gaserzeugungsfluid in den Blutkreislauf (100) getragen wird, und wobei das CO₂-Gas und verbleibende Fluid danach innerhalb des Blutkreislaufs (100) unter Verwendung eines Vorfüllfluids, wie des Dialysefluids für die extrakorporale Therapiebehandlung oder Salzlösung, absorbiert und/oder daraus entfernt werden.

5. Extrakorporales Therapiesystem (10a bis 10c) nach Anspruch 4, wobei das System ausgestaltet ist, um den Druck des Gaserzeugungsfluids, welches das CO₂-Gas trägt, abzusenken, um die Freisetzung des CO₂-Gases zu bewirken.

6. Extrakorporales Therapiesystem (10a bis 10c) nach einem der vorhergehenden Ansprüche, wobei der Blutkreislauf (100) eine be-/entlüftete Kammer (114) einschließt, wodurch mindestens eines der folgenden ermöglicht wird: (i) das CO₂-Gas drückt Luft aus einer Be-/Entlüftung (116) der be-/entlüfteten Kammer (114) oder (ii) ein Vorfüllfluid, wie das Dialysefluid für die extrakorporale Therapiebehandlung oder Salzlösung, drückt das CO₂-Gas aus der Be-/Entlüftung (116).

7. Extrakorporales Therapiesystem (10a bis 10c) nach einem der vorhergehenden Ansprüche, das eine CO₂-Gas-kammer (90) in Fluidkommunikation mit der Dialysefluidzubereitungsstruktur (22, 24, 26, 32, 34, 36, 38, 40, 42, 44, 46, 48) einschließt, wobei die CO₂-Gaskammer (90) so bemessen und angeordnet ist, dass das CO₂-Gas erzeugende Vorfüllfluid gemischt wird, wodurch bewirkt wird, dass CO₂-Gas erzeugt wird.

8. Extrakorporales Therapiesystem (10a bis 10c) nach Anspruch 7, das eine Dosierpumpe (82) einschließt, die positioniert und angeordnet ist, um Bicarbonat und (i) eine Citronensäurelösung, (ii) eine Essigsäurelösung, (iii) eine Milchsäurelösung, (iv) eine Äpfelsäurelösung, (v) eine Salzsäurelösung ("HCl"), (vi) eine Phosphorsäurelösung, (vii) jegliche sonstige biokompatible Säurelösung oder (v) Mischungen davon zu pumpen.

9. Extrakorporales Therapiesystem (10a bis 10c) nach Anspruch 7, das eine CO₂-Gasleitung (86) einschließt, die sich von der CO₂-Gaskammer (90) zu einer Stelle erstreckt, wo das CO₂-Gas in den Blutkreislauf (100) eingebracht werden kann,
und wobei die Stelle eine Leitung in Fluidkommunikation mit einem Anschluss des Blutfilters (102) oder eine Leitung in Fluidkommunikation mit einer Blutleitung (104, 106), die sich von dem Blutfilter (102) erstreckt, einschließt.

10. Extrakorporales Therapiesystem (10a bis 10c) nach Anspruch 7, wobei die CO₂-Gaskammer (90) separat von dem Blutfilter (102) vorliegt.

11. Extrakorporales Therapiesystem (10a bis 10c) nach einem der vorhergehenden Ansprüche, wobei mindestens eines von folgenden zutrifft: (i) eine Ersatzfluidpumpe (96) wird verwendet, um das Gaserzeugungsfluid zu bewegen, während der Blutkreislauf (100) vorgefüllt wird, oder (ii) die Blutpumpe (112) wird in mindestens einer Richtung betrieben, während der Blutkreislauf (100) mit CO₂-Gas vorgefüllt wird, und/oder wobei das Dialysefluid und das Gaserzeugungsfluid aus mindestens einem gemeinsamem Konzentrat (26) gebildet werden.

12. Extrakorporales Therapiesystem (10a bis 10c) nach Anspruch 7, das eine Rezirkulationsleitung (138) in Fluidkommunikation mit der CO₂-Gaskammer (90) einschließt, um das Gaserzeugungsfluid zu durchmischen, um die Freisetzung des CO₂-Gases zu unterstützen.

13. Vorfüllverfahren für ein extrakorporales Therapiesystem, umfassend:
Speichern einer ersten Formel oder eines ersten Sollwerts zum Zubereiten eines Dialysefluids für eine extrakorporale Therapiebehandlung in einem Computerspeicher (18);
Speichern einer zweiten anderen Formel oder eines zweiten anderen Sollwerts zur Zubereitung eines Gaserzeugungsfluids für Kohlendioxid- ("CO₂")-Gasvorfüllung in dem Computerspeicher (18);
Bereitstellen einer Fluidzubereitungsstruktur (22, 24, 26, 32, 34, 36, 38, 40, 42, 44, 46, 48) zur Zubereitung des Gaserzeugungsfluids gemäß der zweiten Formel oder dem zweiten Sollwert;
Zulassen, dass CO₂-Gas aus dem Gaserzeugungsfluid erzeugt wird; und
Vorfüllen des Blutkreislaufs (100) mit dem CO₂-Gas, wobei der Blutkreislauf (100) einen Blutfilter (102) einschließt.

14. Vorfüllverfahren nach Anspruch 13, wobei Vorfüllen des Blutkreislaufs (100) mit dem CO₂-Gas einschließt, dass dem CO₂-Gas das Eintreten in den Blutkreislauf (100) ermöglicht wird,
und wobei Ermöglichen, dass das CO₂-Gas erzeugt wird, und Vorfüllen des Blutkreislaufs (100) mit dem CO₂-Gas einschließt, dass ermöglicht wird, dass das CO₂-Gas aus dem Gaserzeugungsfluid nach dem Eintreten in den Blutkreislauf (100) freigesetzt wird.

15. Vorfüllverfahren nach einem der Ansprüche 13 oder 14,
wobei die zweite andere Formel oder der zweite andere Sollwert zum Zubereiten des Gaserzeugungsfluids beinhaltet: (i) ein Mischverhältnis zwischen Bicarbonat und einer Lösung, die Citronensäure enthält, (ii) ein Mischverhältnis zwischen Bicarbonat und einer Lösung, die Essigsäure enthält, (iii) ein Mischverhältnis zwischen Bicarbonat und einer Lösung, die Milchsäure enthält, (iv) ein Mischverhältnis zwischen Bicarbonat und einer Lösung, die Äpfelsäure enthält, (v) ein Mischverhältnis zwischen Bicarbonat und einer Lösung, die Salzsäure ("HCl") enthält, (vi) ein Mischverhältnis zwischen Bicarbonat und einer Lösung, die Phosphorsäure enthält, (vii) ein Mischverhältnis zwischen Bicarbonat und jeglicher anderen biokompatiblen Säurelösung, oder (v) Gemische davon, und/oder wobei die zweite andere Formel oder der zweite andere Sollwert zum Zubereiten des Gaserzeugungsfluids andere konstituierende Bestandteile oder einen anderen Aufbau der gleichen konstituierenden Bestandteile wie das Dialysefluid für die extrakorporale Therapiebehandlung einschließt.

## Revendications

1. Système de thérapie extracorporelle (10a à 10c) comprenant :
un circuit de fluides de dialyse (30) comprenant une structure de préparation de fluides de dialyse (22, 24, 26, 32, 34, 36, 38, 40, 42, 44, 46, 48) configurée pour préparer un fluide de dialyse pour un traitement de thérapie extracorporelle ;
un circuit de sang (100) comprenant un filtre à sang (102) pour une utilisation pendant le traitement de thérapie extracorporelle ;
une pompe à sang (112) exploitable pour pomper du sang à travers le circuit de sang (100) et le filtre à sang (102) ; et
une unité de commande (20) exploitable avec la structure de préparation de fluides de dialyse (22, 24, 26, 32, 34, 36, 38, 40, 42, 44, 46, 48) et la pompe à sang (112),
ledit système de thérapie extracorporelle étant **caractérisé par le fait que** l'unité de commande (20) est programmée pour préparer un fluide de génération de gaz différent du fluide de dialyse pour le traitement de thérapie extracorporelle, le fluide de génération de gaz générant du gaz de dioxyde de carbone (« CO₂ »), et le gaz de CO₂ étant utilisé pour amorcé le circuit de sang (100) comprenant le filtre à sang (102).

2. Système de thérapie extracorporelle (10a à 10c) selon la revendication 1, l'unité de commande (20) étant en outre programmée pour absorber et/ou rincer le gaz de CO₂ du circuit de sang (100) en utilisant un fluide d'amorçage, tel que le fluide de dialyse pour le traitement de thérapie extracorporelle ou une solution saline, amorçant ainsi davantage le circuit de sang (100).

3. Système de thérapie extracorporelle (10a à 10c) selon les revendications 1 ou 2, le gaz de CO₂ étant généré dans le circuit de fluides de dialyse (30) et délivré du circuit de fluides de dialyse (30) au circuit de sang (100).

4. Système de thérapie extracorporelle (10a à 10c) selon la revendication 1, le gaz de CO₂ étant transporté par le fluide de génération de gaz dans le circuit de sang (100), et le gaz de CO₂ et le fluide restant étant ensuite absorbés dans et/ou éliminés du circuit de sang (100) en utilisant un fluide d'amorçage, tel que le fluide de dialyse pour le traitement de thérapie extracorporelle ou une solution saline.

5. Système de thérapie extracorporelle (10a à 10c) selon la revendication 4, le système étant configuré pour baisser la pression du fluide de génération de gaz transportant le gaz de CO₂ pour causer la libération du gaz de CO₂.

6. Système de thérapie extracorporelle (10a à 10c) selon l'une quelconque des revendications précédentes, le circuit de sang (100) comprenant un compartiment ventilé (114) permettant au moins l'un parmi (i) la poussée par le gaz de CO₂ d'air hors d'un conduit d'aération (116) du compartiment ventilé (114) ou (ii) la poussée par un fluide d'amorçage, tel que le fluide de dialyse pour le traitement de thérapie extracorporelle ou une solution saline, de gaz de CO₂ hors du conduit d'aération (116).

7. Système de thérapie extracorporelle (10a à 10c) selon l'une quelconque des revendications précédentes, qui comprend un compartiment de gaz de CO₂ (90) en communication fluidique avec la structure de préparation de fluides de dialyse (22, 24, 26, 32, 34, 36, 38, 40, 42, 44, 46, 48), le compartiment de gaz de CO₂ (90) étant dimensionné et agencé pour mélanger le fluide d'amorçage de génération de gaz de CO₂, entraînant la génération de gaz de CO₂.

8. Système de thérapie extracorporelle (10a à 10c) selon la revendication 7, qui comprend une pompe de dosage (82) positionnée et agencée pour pomper du bicarbonate et (i) une solution d'acide citrique, (ii) une solution d'acide acétique, (iii) une solution d'acide lactique, (iv) une solution d'acide malique, (v) une solution d'acide chlorhydrique (« HCl »), (vi) une solution d'acide phosphorique, (vii) une quelconque autre solution d'acide biocompatible, ou (v) des mélanges correspondants.

9. Système de thérapie extracorporelle (10a à 10c) selon la revendication 7, qui comprend une ligne de gaz de CO₂ (86) s'étendant depuis le compartiment de gaz de CO₂ (90) vers un emplacement où le gaz de CO₂ peut être introduit dans le circuit de sang (100), et l'emplacement comprenant une ligne en communication fluidique avec un port du filtre à sang (102) ou une ligne en communication fluidique avec une ligne de sang (104, 106) s'étendant depuis le filtre à sang (102).

10. Système de thérapie extracorporelle (10a à 10c) selon la revendication 7, le compartiment de gaz de CO₂ (90) étant distinct du filtre à sang (102).

11. Système de thérapie extracorporelle (10a à 10c) selon l'une quelconque des revendications précédentes, au moins l'une parmi (i) une pompe de fluide de remplacement (96) étant utilisée pour déplacer le fluide de génération de gaz tout en amorçant le circuit de sang (100) ou (ii) la pompe à sang (112) étant exploitée dans au moins une direction tout en amorçant le circuit de sang (100) avec du gaz de CO₂, et/ou le fluide de dialyse et le fluide de génération de gaz étant formés à partir d'au moins un concentré commun (26).

12. Système de thérapie extracorporelle (10a à 10c) selon la revendication 7, qui comprend une ligne de recirculation (138) en communication fluidique avec le compartiment de gaz de CO₂ (90) pour agiter le fluide de génération de gaz pour aider la libération du gaz de CO₂.

13. Procédé d'amorçage pour un système de thérapie extracorporelle comprenant :
le stockage dans une mémoire d'ordinateur (18) d'une première formule ou consigne pour la préparation d'un fluide de dialyse pour un traitement de thérapie extracorporelle ;
le stockage dans la mémoire d'ordinateur (18) d'une deuxième formule ou consigne différente pour la préparation d'un fluide de génération de gaz pour l'amorçage de gaz de dioxyde de carbone (« CO₂ ») ;
la fourniture d'une structure de préparation de fluides (22, 24, 26, 32, 34, 36, 38, 40, 42, 44, 46, 48) pour préparer le fluide de génération de gaz selon la deuxième formule ou consigne ;
le fait de laisser du gaz de CO₂ être généré à partir du fluide de génération de gaz ; et
l'amorçage d'un circuit de sang (100) avec le gaz de CO₂, le circuit de sang (100) comprenant un filtre à sang (102).

14. Procédé d'amorçage selon la revendication 13, l'amorçage du circuit de sang (100) avec le gaz de CO₂ comprenant le fait de permettre l'entrée du gaz de CO₂ dans le circuit de sang (100),
et le fait de laisser le gaz de CO₂ être généré et l'amorçage du circuit de sang (100) avec le gaz de CO₂ comprenant le fait de permettre au gaz de CO₂ d'être libéré du fluide de génération de gaz après l'entrée dans le circuit de sang (100).

15. Procédé d'amorçage selon l'une quelconque des revendications 13 ou 14,
la deuxième formule ou consigne différente pour la préparation du fluide de génération de gaz comprenant (i) un rapport de mélange entre du bicarbonate et une solution contenant de l'acide citrique, (ii) un rapport de mélange entre du bicarbonate et une solution contenant de l'acide acétique, (iii) un rapport de mélange entre du bicarbonate et une solution contenant de l'acide lactique, (iv) un rapport de mélange entre du bicarbonate et une solution contenant de l'acide malique, (v) un rapport de mélange entre du bicarbonate et une solution contenant de l'acide chlorhydrique (« HCl »), (vi) un rapport de mélange entre du bicarbonate et une solution contenant de l'acide phosphorique, (vii) un rapport de mélange entre du bicarbonate et une quelconque autre solution d'acide biocompatible, ou (v) des mélanges correspondants, et/ou la deuxième formule ou consigne différente pour la préparation du fluide de génération de gaz comprenant une consistance différente ou une composition différente d'une même consistance que le fluide de dialyse pour le traitement de thérapie extracorporelle.
